# EUROPEAN PATENT SPECIFICATION

(11) **EP 1 954 810 B1**
(45) Date of publication and mention of the grant of the patent: **28.12.2011**
(21) Application number: 06809100.8
(22) Date of filing: 23.10.2006
(51) Int. Cl.: C12N 15/55, C12N 15/11, C12N 9/16, C12Q 1/68, C12P 19/34

(54) **GENETIC VARIANTS OF HUMAN INOSITOL POLYPHOSPHATE-4-PHOSPHATASE, TYPE I (INPP4A) USEFUL FOR PREDICTION AND THERAPY OF IMMUNOLOGICAL DISORDERS**
GENETISCHE VARIANTEN VON MENSCHLICHER INOSITOLPOLYPHOSPHAT-4-PHOSPHATASE TYP I (INPP4A) MIT EIGNUNG ZUR VORHERSAGE UND THERAPIE IMMUNOLOGISCHER ERKRANKUNGEN
VARIANTES GÉNÉTIQUES DE POLYPHOSPHATE-4-PHOSPHATASE INOSITOL HUMAIN, DE TYPE I (INPP4A) SERVANT A LA PREDICTION ET LA THERAPIE DE TROUBLES IMMUNOLOGIQUES

(30) Priority: 25.10.2005 IN DE15362005
(43) Date of publication of application: 13.08.2008
(73) Proprietor: Council of Scientific and Industrial Research, New Delhi 110 001 (IN)
(72) Inventor: GHOSH, Balaram, New Delhi 110 007 (IN); SHARMA, Mamta, New Delhi 110 007 (IN); BATRA, Jyotsna, New Delhi 100 007 (IN)
(74) Representative: Icely, Dominic Michael
(86) International application number: PCT/IB2006/002963
(87) International publication number: WO 2007/049118

(56) References cited:
- DIB ET AL: "A comprehensive genetic map of the human genome based on 5264 microsatellites" NATURE, NATURE PUBLISHING GROUP, LONDON, GB, vol. 380, 14 March 1996 (1996-03-14), pages 152-154, XP002095715 ISSN: 0028-0836
- DATABASE SNP NCBI, Bethesda, USA; 7 August 2002 (2002-08-07), XP002421705 Database accession no. rs3768712
- DATABASE SNP NCBI, Bethesda, USA; 5 September 2001 (2001-09-05), XP002421702 Database accession no. rs2278206
- DATABASE SNP NCBI, Bethesda, USA; 5 November 2003 (2003-11-05), XP002421701 Database accession no. rs10201079
- DATABASE SNP NCBI, Bethesda, USA; 5 September 2001 (2001-09-05), XP002421703 Database accession no. rs2278208
- DATABASE SNP NCBI, Bethesda, USA; 7 August 2002 (2002-08-07), XP002421704 Database accession no. rs3769710
- VYAS PARESH ET AL: "Inositol polyphosphate 4-phosphatase type I regulates cell growth downstream of transcription factor GATA-1" PROCEEDINGS OF THE NATIONAL ACADEMY OF SCIENCES OF THE UNITED STATES OF AMERICA, vol. 97, no. 25, 5 December 2000 (2000-12-05), pages 13696-13701, XP002421693 ISSN: 0027-8424
- SHEARN COLIN T ET AL: "Identification of a novel spliceoform of inositol polyphosphate 4-phosphatase type Ialpha expressed in human platelets: Structure of human inositol polyphosphate 4-phosphatase type I gene" BIOCHEMICAL AND BIOPHYSICAL RESEARCH COMMUNICATIONS, vol. 286, no. 1, 10 August 2001 (2001-08-10), pages 119-125, XP002421694 ISSN: 0006-291X
- NORRIS F ANDERSON ET AL: "The isolation and characterization of cDNA encoding human and rat brain inositol polyphosphate 4-phosphatase" JOURNAL OF BIOLOGICAL CHEMISTRY, vol. 270, no. 27, 1995, pages 16128-16133, XP002421695 ISSN: 0021-9258

### SEQUENCE LISTING

<110> COUNCIL OF SCIENTIFIC AND INDUSTRIAL RESEARCH
<120> GENETIC VARIANTS OF HUMAN INOSITOL POLYPHOSPHATE-4-PHOSPHATASE, TYPE I (INPP4A) USEFUL FOR PREDICTION AND THERAPY OF IMMUNOLOGICAL DISORDERS
<130> PCT0657
<150> 1536DEL2005
   <151> 2005-10-25
<160> 26
<170> PatentIn version 3.3
<210> 1
   <211> 401
   <212> DNA
   <213> Homo sapiens
<400> 1
   ggggcaagtg gcgatagggc tttctacata cacacacaca cacacacaca cacacacaca      60
   cacacacaca caatgatgga agtaaattag agggacatag caaccaacct gaaagagctc     120
   ccaatggcca aaactgaagc catttgaaaa acaaaatttt taaatttagt attagactat     180
   atcccaaagt ataaaagtcc atgagtctat gccataaatg aatgattgaa taaataaatt     240
   aatgagggag aaaggacaca tattttttac agaaggattc cagatcatat atgtagatta    300
   tttgatactt tccaccccag gatgtaaagc cttattgatg gatcttgcac tcagaactgg     360
   agccaaggat ccagcagcct ctacactgcc atgggggaac a                         401
<210> 2
   <211> 240
   <212> DNA
   <213> Homo sapiens
<400> 2
   gggctggagg gggtggagta gggagtgttg tgaaagtttc aagcacagag gactgaatgg      60
   aagcctcaca atgcaaccta aggactttgg attcttgctt ttgagtacat gcactgtgtg     120
   tgtgtgtgtg tgtgtgtgtg tgtgtgtgtg tgtgtgtgtg tgtaaatggt gtagtttgtt     180
   ggctgtcagc cttactagga ggctggtttg gagcctgttg ccttagctct gtggggtgat     240
<210> 3
   <211> 159
   <212> DNA
   <213> Homo sapiens
<400> 3
   tcacccagta ccagaccatc atcctcacat accaggagaa cctgaccgac ctccatcagt     60
   acagaggtgg gtgcaccccc atgctgtcac cacacacgcg tgcgcacaca cacacacaca    120
   cactctcact ctcactcagt cactccctct gctttccca                           159
<210> 4
   <211> 1036
   <212> DNA
   <213> Homo Sapiens
<400> 4
   tgcagcagcc cttagtttgt ttagagaact tgattcagga aaaaggaacc atctttggta     60
   aaatgagagg gatcatgtaa gggaaagggc tttgagaaaa atatttagtt acgtcatgtg    120
   tcagagttaa ctctagttgg gttagagtta aatgtacaaa atagacatca ttctttttaa    180
   aggagctaaa ggcggctgat gattgaacag atagagcagg aaaggacttc acaggcacga    240
   ggacagggca aggaaatcac aaaggaaatg gtcagtagat gggaccagac aaaaatttag    300
   aacctgatta agtgagacct tgtaaataga aagcctgaag ttccagggtg tccccaggtt    360
   ccctggatga atcagatgag cccaggccac catcagccac ccttcgggat gtcagggtaa    420
   agctgactga ggctggggta gcatcagctg tgtcatatca ggattcaagg gcttcaggaa    480
   gccctggctg gattccatta cttggagtcc aggcttaggc cttgggctaa tataagcctc    540
   aaggtggggt gggaaggaac agaaaagggc ccagggccag aacaaggtgt tctgtagtcc    600
   tcactctgac acgtgtcccc ctcaccctct gggcaggggt gagcacaggc actggcagca    660
   gctcaggaac agcattggct cctcctcagg gaggccccct ctgacttcac cagcacgtcc    720
   acacttccct gtggtgcgtg tccgttcaga cacagccctc cactgtgccc catggtttcc    780
   ttgtctgtct cgcctgagag gagccctgtg tgcacagtga atgctgactg actggtcagt    840
   cagctcggcc ctgctgcccc caggaccctg atgacccagt acggctaggg ctttgtattt    900
   cagcagaaaa gaagcacagt tgcagcacca ctcattaaag catgttgtgc atcacaggtc    960
   tgcagagagt gaccgtgtag gtaacatcac cgtgattggc tggcagatgg aggagaagtc   1020
   agaccaacgg ccccct                                                   1036
<210> 5
   <211> 961
   <212> DNA
   <213> Homo Sapiens
<400> 5
   atggcgaggg ctgtgaggat gtcttcccct gtgcaggcag ctgcaccagc aagaaaggta    60
   acccggacag ccacgcctac tggatcagac cagaagaccc cttctgtgat gtcccctcct   120
   caccatgccc ctccaccatg ccctccactg catgccatcc tcatctgacc acacgtgcgt   180
   atgcatccat gcttcccata tgctgcctcc atttcacctt gtgctgcttc catttgtctg   240
   tgtctctgac tccatgccct cctcccccat gcagactgca gggagggctg ccaggtggag   300
   cgggggcgtc ccagtgcatg gagcagcagg cagtgagagc ggagtctccg gttgcctttc   360
   catgtaactg gagcccatgt gtgattctgc atctctgcca gtcagaatct cagggtcctc   420
   ttgagataca ccaccatggc tataagggtt gggctcctgg gcttttcagg tttgtaaagc   480
   aatctgagaa cctgaaggag gccaggatgc agatgccttt ctgatgtgat tgtctatggc   540
   attaggaatt ggtagaatga aaggggaaag gggaattgtt cagggacaca gctggatgag   600
   agatattggc aggtccagtg gacatgtcta aaagctggtt ggcattggct gggcactctc   660
   ttatgcagca gaggcatgtt gccttgggac ttggtaacac agagaatggg accctgttcc   720
   tgggcttctg gtggtctgag tcaggtggtt tggcaggacc cttcagggta ggacactggc   780
   atgaagcagc atgcttcaga acagtctcct gtctgcagag acacagcata ccaggacact   840
   gtcctgcaga tggccttaaa gctgggaaac agtccctgca actgagatgt ctccgccttt   900
   tcctgtaaga gccatagagt tacaagctag gcagctgaga cttgcccaca gacctagctt   960
   c                                                                   961
<210> 6
   <211> 1707
   <212> DNA
   <213> Homo Sapiens
<400> 6
   ccttggctgc atgggttctc acgttgtcca ccgaatccca ggcatcccct ggggataagg     60
   ggttgtgggg cagcagtgcc agccactcag gtactgagtc cttccatccc cagtttgagc    120
   agagctgctc tccttttcag gctcttattt ctttttgttt gtgtgtatga ataaagggtc    180
   cctggctgaa tagctggaat atctttagtc aggtctaagc ctatatctca tggatgggga    240
   gacccagcag tgcaccacaa gtggctggtg tccctctggg atggaagcct ggcactgcag    300
   tgccctcttg ggcaaggatg catcactggc catccctgcc tgccttccag aacctcacgt    360
   tagctctgga tgcagcatct atgaatttaa agctttttga aacctatagt tccagcttcc    420
   gtcatgtgtt tgggtcatga actctacagc ccacagaatt tagtgcacat accaagttca    480
   ctcttgcttt gtcagttaca actttacaaa tttgattaat ttactctcca ttagcttagg    540
   gtctgcttat ctgcttaggg cctgttattt ttttttcttt ctttttgttg gtctgttctc     600
   agaagaagcc tattctcctt ctgattgtct tgcctgaaca ttgggtcttt tgttttgcct    660
   ttccctagac gaggttctta gcactgtacc atgttcgtgt ctagtgatga tcctggtagt    720
   gactggcatg ctctttggct ttgctggcct gacaagcagg tgttgtgcca tcttcaggag    780
   ttgatgagac actgatattt ttctggcata caacagatac ccggagccca ccatcatatc    840
   acttggatga tgtttcatta aataccagcc ttggtgtcat agtgcagtat ctgatacggt    900
   gcatagtcct ttatctgaag cccctgtggc cagatgcttt ggaatgcaca ggttttcaga    960
   ttttagaaag tactgtggta cacagttggt gtttacttat gtaagtaata tggtgcccat   1020
   cgcatatgtg taccatggta tacatacagg atttaacatc cccgggggag cctgagacag   1080
   cactctgcaa ccaagctcat attattgcca cactgaaaag tatgaatatt tacataaaat   1140
   gggataagta aagattgcaa gtagtcttat atcagctcag gtctggttgt gctgccaaat   1200
   gagattgcca cagatttaca tgaaaaaatt accttgtttc agagcttgat ggatttggtg   1260
   actgtgtgcc taggattgta tctagaggtg cagggaaaat gccctgaaat ctccccatgg   1320
   ggagaggagg tgcactcagg tggccttttc ccagtccaga ggctgccctg ccccctactc  1380
   tgtgagccat tcagttgtga ggagggaagt gtgctggcag taactctgct accgggaaag   1440
   tatggcggga ggaggcctgt ggggctggtg gtcaggaggt ggccaaaggg cagtgcaact   1500
   tctgagctgc tcccgtctgt tcaggtgaaa gaggagagta gagatagtag gtctgttccc   1560
   taccttccag ggagacctag agacctggcc caggactgtg ggcatcctga gagtggctct   1620
   ggcttcccca gtgggtcata tttgccgccc tctttccctg agggatcagc agtgaagggc   1680
   tgaacgtgat aagagggccc aggcatg                                       1707
<210> 7
   <211> 18
   <212> DNA
   <213> Artificial
<220>
   <223> Forward primer for amplifying Sequence ID: 1
<400> 7
   ggggcaagtg gcgatagg 18
<210> 8
   <211> 20
   <212> DNA
   <213> Artificial
<220>
   <223> Reverse primer for amplifying sequence ID: 1
<400> 8
   tgttccccca tggcagtgta 20
<210> 9
   <211> 21
   <212> DNA
   <213> Artificial
<220>
   <223> Forward primer for amplifying sequence ID: 2
<400> 9
   gggctggagg gggtggagta g 21
<210> 10
   <211> 25
   <212> DNA
   <213> Artificial
<220>
   <223> Reverse primer for amplifying Sequence ID: 2
<400> 10
   atcaccccac agagctaagg caaca 25
<210> 11
   <211> 23
   <212> DNA
   <213> Artificial
<220>
   <223> Forward primer for amplifying Sequence ID: 3
<400> 11
   tcacccagta ccagaccatc atc 23
<210> 12
   <211> 22
   <212> DNA
   <213> Artificial
<220>
   <223> Reverse primer for amplifying sequence ID: 3
<400> 12
   tgggaaagca gagggagtga ct 22
<210> 13
   <211> 24
   <212> DNA
   <213> Artificial
<220>
   <223> Forward primer for amplifying Sequence ID: 4
<400> 13
   tgcagcagcc cttagtttgt ttag 24
<210> 14
   <211> 18
   <212> DNA
   <213> Artificial
<220>
   <223> Reverse primer for amplifying Sequence ID: 4
<400> 14
   agggggccgt tggtctga 18
<210> 15
   <211> 23
   <212> DNA
   <213> Artificial
<220>
   <223> Forward primer for amplifying Sequence ID: 5
<400> 15
   atggcgaggg ctgtgaggat gtc 23
<210> 16
   <211> 24
   <212> DNA
   <213> Artificial
<220>
   <223> Reverse primer for amplifying Sequence ID: 5
<400> 16
   gaagctaggt ctgtgggcaa gtct 24
<210> 17
   <211> 21
   <212> DNA
   <213> Artificial
<220>
   <223> Forward primer for amplifying Sequence ID: 6
<400> 17
   ccttggctgc atgggttctc a 21
<210> 18
   <211> 21
   <212> DNA
   <213> Artificial
<220>
   <223> Reverse primer for amplifying sequence ID: 6
<400> 18
   catgcctggg ccctcttatc a 21
<210> 19
   <211> 19
   <212> DNA
   <213> Artificial
<220>
   <223> Snapshot primer for SNP 75 A/T (rs3769712) in Sequence ID: 4
<400> 19
   caaagccctt tcccttaca 19
<210> 20
   <211> 22
   <212> DNA
   <213> Artificial
<220>
   <223> Snapshot primer for 388 C/T polymorphism (rs3769710) in Sequence ID: 4
<400> 20
   atcccgaagg gtggctgatg gt 22
<210> 21
   <211> 25
   <212> DNA
   <213> Artificial
<220>
   <223> Snapshot primer for 861 C/T (rs2278208) in Sequence ID: 4
<400> 21
   agccgtactg ggtcatcagg gtcct 25
<210> 22
   <211> 20
   <212> DNA
   <213> Artificial
<220>
   <223> Snapshot primer for C/T polymorphism (rs2278206) at nucleotide 147 in Sequence ID: 5
<400> 22
   cagatgagga tggcatgcag 20
<210> 23
   <211> 18
   <212> DNA
   <213> Artificial
<220>
   <223> Snapshot primer for C/T polymorphism (rs10201079) at nucleotide 1221 in sequence ID: 6
<400> 23
   gattgccaca gatttaca 18
<210> 24
   <211> 816
   <212> DNA
   <213> Artificial
<220>
   <223> CDNA sequence containing splice variant region
<400> 24
   gggcgccagc agcacactgc caaggtttta agtcaggagg tctccgcaaa aagctgcaca      60
   aatttgaaga gaccaagaaa cattttgagg agtgttgtac atcatctggc tgccagtcca     120
   taatctacat accccaggat gttgtcagag ccaaggagat catcgcccag atcaacaccc     180
   tgaaaaccca agtgagttac tacgcagagc ggctgtcaag ggcagccaag gacaggtctg     240
   ccactggcct tgagaggaca ctcgccatct tggcagacaa gacacggcag ctggtcacgg     300
   tctgcgactg caagctcctg gccaactcca tccatgggct gaacgctgca cggcctgact     360
   acattgcctc caaggcctct cccacttcga ctgaggagga gcaggtgatg cttagaaatg     420
   accaggacac cctcatggcc cggtggacag ggagaaacag ccgatcttcc ctgcaggtgg     480
   actggcacga ggaggagtgg gagaaagtgt ggctgaacgt ggacaagagc ctagagtgca     540
   tcattcagcg tgtggacaag ctgctgcaga aggagcggct gcatggcgag ggctgtgagg     600
   atgtcttccc ctgtgcaggc agctgcacca gcaagaaagg taacccggac agccacgcct     660
   actggatcag accagaagac cccttctgtg atgtcccctc ctcaccatgc ccctccacca     720
   tgccctctac tgcatgccat cctcatctga ccacacattg cagtccccct cctgaagagt     780
   ccagcccagg tgaatggagt gaggcccttt acccgc                               816
<210> 25
   <211> 19
   <212> DNA
   <213> Artificial
<220>
   <223> Forward primer for amplifying CDNA sequence containing splice variant region
<400> 25
   ggggcgccag cagcacact 19
<210> 26
   <211> 24
   <212> DNA
   <213> Artificial
<220>
   <223> Reverse primer for amplifying CDNA sequence containing splice variant region
<400> 26
   gcgggtaaag ggcctcactc catt 24

## Description

### FIELD OF THE INVENTION

The present invention relates to genetic variants including splice variants of the human Inositol polyphosphate 4-phosphatase (INPP4A) gene useful for the prediction of susceptibility to asthma. More particularly, the invention provides primers and methods suitable for the detection of haplotypes of allelic variants of the INPP4A gene for the prediction of an individual's susceptibility to asthma.

### BACKGROUND AND PRIOR ART REFERENCES OF THE PRESENT INVENTION

Asthma is a common chronic airway disease, with considerable heterogeneity both in its phenotype and in the underlying pathophysiology. It affects 15-18% of the world's population. Both intrinsic and extrinsic cases of asthma are known. Intrinsic asthma is mainly childhood disorder though the age of onset can vary and is seen to be 35-45 years in the general population; whereas extrinsic asthma is observed where the age of onset is above 45 yr. and is mainly due to the age induced changes in the lung function. Recent studies have demonstrated that airway inflammation is a principal feature in the pathophysiology of asthma (Gem et al. 1999). The disorder is multifactorial (in both initiation and progression) because of the involvement of numerous resident and recruited inflammatory cells. T cells and IgE-mediated responses are known to be a key factor in the allergic response (Elias et al. 2003). Asthma is a T helper type 2 (Th2) mediated disorder with cytokines such as interleukin-4, interleukin-5, interleukin-13, implicated in the deviation of the immune system towards atopicity. Increased levels of these cytokines lead to elevated total serum IgE levels, eosinophil recruitment, and bronchial hyper-responsiveness that ultimately culminate in asthma pathogenesis. These interleukins are also known to interact and stimulate the alveolar cells and bronchial smooth muscle cells resulting in the clinical phenotypes of bronchial hyper-responsiveness (Barnes P. J., Respir. Res.2:64-5, 1999). Gene-gene and gene-environment interactions have been implicated in the development of asthma (Tay et al, Asian Pac J Allergy Immunol 17:239-42, 1999; Bleecker ER, Am J Respir Crit Care Med 156:S113-6,1997; Cookson W, Nature 402:B5-11, 1999).

Inflammation and airway remodeling are characteristic features of atopic asthma. Various types of cells viz: eosinophils, mast cells, and T lymphocytes migrate to the lungs and mediate the inflammation process at the site (Barnes PJ, 1992). In addition to these cells, platelets play a key role in this allergic inflammatory process, because they are a rich source of a wide range of biologically active materials capable of inducing or augmenting allergic inflammatory responses (Herd CM, 1994, Klinger, 1995). Such materials have been demonstrated to be preformed mediators stored in a-granules, which are chemokines such as platelet factor 4 (PF4) and regulated upon activation in normal T cells expressed and presumably secreted (RANTES) (Herd CM, 1994, Klinger, 1995). These chemokines are released from platelets after stimulation with potent anaphylactic mediators such as platelet activating factor (PAF) (Herd CM, 1994, Kameyoshi Y, 1992) and cause eosinophilic chemotaxis (Kameyoshi Y, 1992), providing additional evidence for a contribution of platelets to bronchial asthma. Studies in mouse model of allergic inflammation suggest the role of platelets in airway remodeling (Simon C et al, 1994). Platelet activation has been found to be associated with inactivation of a magnesium independent enzyme Inositol polyphosphate 4-phosphatase (INPP4A, EC 3.1.3.66) (Norris FA, 1997). Stimulation of human platelets with thrombin or calcium ionophore results in inactivation of INPP4A by proteolytic cleavage by calcium dependent protease calpain (Norris FA et al, 1997). The enzyme INPP4A catalyzes the hydrolysis of the 4-position phosphate of inositol 3,4-bisphosphate and inositol 1,3,4-trisphosphate. It also catalyzes, at a much higher rate, the hydrolysis of the 4-position phosphate of phosphatidylinositol 3,4-bisphosphate generated by the phosphorylation at the D-3 position of inositol lipids by Phosphoinositide 3-kinases (PI3Ks). Thus inactivation of INPP4A is associated with calcium/aggregation dependent accumulation of PtdIns (3,4) P2 characteristic of stimulated human platelets. Norris et al. (1995) noted that INPP4A is also implicated in mitogenesis mediated by PDGF receptor, the oxidative burst of neutrophils, translocation of the glucose transporter to the plasma membrane. Vyas P et al 2000 found that INPP4A regulates cell proliferation downstream of GATA-1 transcription factor. GATA-1⁻ megakaryocytes (precursor of platelets) were found to be deficient in this enzyme and showed hyperproliferation. Reintroduction of INPP4A into GATA-1⁻ megakaryocytes significantly retarded cell growth, suggesting the role of this enzyme in cell proliferation. When differential gene expression was compared between lungs of ovalbumin sensitized/ challenged A/J mice versus control mice, various genes were found to be differentially expressed (Gene Expression Omnibus Database (GEO), GDS 349). Among these differentially expressed genes, INPP4A was also found to be modulated in the lungs of sensitized mice.

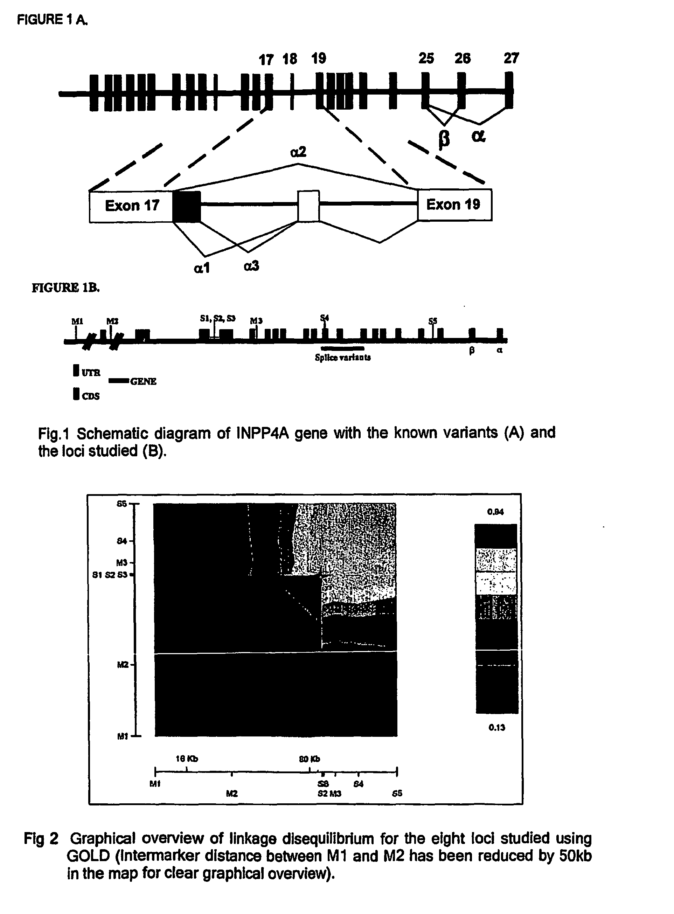

The INPP4A gene encodes two protein isoforms α and β varying in their carboxy terminus and various splice variants are also known in the exon 17, 18 and 19 region of this gene (NT022171), earlier designated as exon 15, 16 and 17 (Shearn CT et al, 2001). Two more exons encoding 5' UTR have been annotated in the contig NT022171. There are 3 splice forms viz; α 1, 2 and 3 reported in the exon 17-19 region as shown in Figure 1A.

INPP4 α 1 encodes a 106 kDa form of the major enzyme expressed in human, rat and mouse brain (Norris FA et al, 1995; Vyas P et al, 2000). INPP4 α 2 encodes a 102 kDa form that is minor species expressed in rat and mouse brain (Norris FA et al, 1995). The α 3 isoform results from the use of an alternative 5'- GU splice donor site during the excision of intron 17 and extends the exon 17 by 120 bp and encodes a 110 kDa protein expressed as the major form in human platelets (Shearn CT et al, 2001) . This extended exon 17 region contains three repeats spaced seven bases apart with the nucleotide sequence CCCCTYCW where Y represents C or T and W represents A or T. Exon 18 also contains a sequence with this consensus. These consensus pyrimidine rich elements represent recognition sites for splicing factors that regulate the tissue specific alternative splicing of exons 17 and 18. The extended exon 17 encodes a 40 amino acid domain, which contains PEST sequence. PEST sequences are rich in proline, serine, glutamate/aspartate and threonine residues and the proteins containing such sequences are rapidly degraded by the calpain family of proteases (Rogers S et al, 1986; Rechsteiner M and Rogers S 1996).

INPP4A enzyme containing PEST sequences also is rapidly degraded by calpain family of proteases, which act on proteins containing PEST sequences (Norris FA et al, 1997). As the stimulation of platelets is associated with asthma, which in turn correlated with inactivation of the *INPP4A* enzyme, we hypothesized that gene variants of *INPP4A* could be associated with immunological disorders including asthma. However, no studies have been done till date to study the genetic role of INPP4A in immunological disorders including atopic asthma.

### Novelty of the invention:

The inventors for the first time have identified INPP4A gene variants associated with asthma in humans, including +92031 A/T (S1), +92344 C/T (S2), +92817 C/T (S3), +110832 A/G (S4), +131237 C/T (S5) single nucleotide polymorphisms and D2S2311 (M1), D2S2187 (M2) and +99095 CA (M3) repeat in the intronic and exonic and flanking regions of the gene.

Thus, in one aspect of the invention, there is provided an *in vitro* method of predicting susceptibility of an individual to asthma which comprises determining whether said individual possesses a haplotype of genetic variants, said variants being selected from genetic variants of the human Inositol polyphosphate 4-phosphatase (INPP4A) gene and the CA dinucleotide repeat at the M1 locus located 44.7 kb upstream of the start site of said gene, wherein said haplotype is positively-associated or negatively-associated with asthma occurrence.

Also provided are specific primers for detection of single nucleotide polymorphisms and specific repetitive sequences in and around the INPP4A gene as defined by SEQ. ID Nos 7-23.

### SUMMARY OF THE INVENTION

Atopic asthma is a chronic, inflammatory lung disease characterized by recurrent breathing problems in response to an allergen. Platelets play an important role in this allergic inflammatory process, by releasing preformed mediators like platelet factor 4 (PF4) and regulated upon activation in normal T cells expressed and secreted (RANTES) upon activation causing eosinophil chemotaxis. The present invention relates to allelic variants of the human *Inositol polyphosphate 4-phosphatase (INPP4A)* gene and splice variants of the coding sequence, which encodes *INPP4A* enzyme known to be an important regulator of platelet activation; and provides primers and methods suitable for the detection of these allelic variants as noted above for prediction of an individual's susceptibility, to asthma.

### DETAILED DESCRIPTION OF THE INVENTION

INPP4A has been identified as a candidate gene for finding association with immunological disorders, particularly asthma as several polymorphisms in this gene were found to be associated with atopic asthma in case-control and family studies.

The INPP4A gene encodes a magnesium independent enzyme Inositol polyphosphate 4-phosphatase, which catalyzes the hydrolysis of the 4-position phosphate of inositol 3,4-bisphosphate and inositol 1,3,4-trisphosphate. It also catalyzes, at a much higher rate, the hydrolysis of the 4-position phosphate of phosphatidylinositol 3,4-bisphosphate generated by the phosphorylation at the D-3 position of inositol lipids by Phosphoinositide 3-kinases (PI3Ks). The INPP4A gene is expressed in humans in various tissues viz. Bladder, Blood, Bone, Bone Marrow, Brain, Cervix, Colon, Eye, Heart, Kidney, Liver, Lung, Lymph Node, Mammary Gland, Muscle, Ovary, Pancreas, Placenta, Prostate, Skin, Soft Tissue, Spleen, Stomach, Testis, Thymus, Uterus (Expression profile from UniGene Cluster Hs.469386). Norris et al. (1995) noted that the latter activity of INPP4A is implicated in mitogenesis mediated by PDGF receptor, the oxidative burst of neutrophils, translocation of the glucose transporter to the plasma membrane and platelet aggregation. Stimulation of human platelets with thrombin or calcium ionophore results in inactivation of INPP4A by proteolytic cleavage by calcium dependent protease calpain (Norris FA et al, 1997). This inactivation of INPP4A is associated with calcium/aggregation dependent accumulation of PtdIns(3,4)P2 characteristic of stimulated human platelets. Thus, INPP4A is an important enzyme involved in regulation of this lipid second messenger in platelets. The platelets are known to be important players in asthma pathogenesis; many studies in the nineties have shown the importance of these cells in asthma. Platelets were found to be necessary for airway remodeling in mouse model of asthma (Simon C et al, 2004) and promote eosinophil adhesion to endothelium in asthmatic individuals (Ulfman LH et al, 2003).

The present application deals with D2S2311; a microsatellite marker, 44.7 kb upstream of the gene, dinucleotide polymorphic repeat at nucleotide 99095 to 99127, which is in the intron 11 of the *INPP4A* gene (GenBank accession no. NT_022171). The first three SNPs (rs3769712, rs3769710, rs2278208), as shown in Figure 1B, are 92031 bp, 92344 bp and 92817 bp downstream of the gene start site respectively and lie in intron 7 of the gene. The fourth non-synonymous SNP (rs2278206) is situated 110832 bp downstream of the human *INPP4A* gene in alternatively spliced exon 17 and represents a A to G transition (Thr to Ala). The fifth SNP rs10201079 is a C to T transition, situated 131237 bases downstream of the *INPP4A* gene start site in the intron 24.

The results of the present study provide very unique results. In addition to identifying, genotyping and establishing a positive association of the microsatellite D2S2311, 44.7 kb upstream of the gene with asthma, the inventors have found an association of the known +92031 A/T, +110832 A/G, +131237 C/T single nucleotide polymorphisms and a dinucleotide repeat at +99095 in the Indian population. All the SNPs and microsatellite repeats have been validated in Indian population for the first time.

The present invention has identified the genetic variants, which exist in any type of population in the world irrespective of its origin, community, colour, geographical location or ethnicity. The inventors have compared allele and genotype frequencies of D2S2311 repeat polymorphism 44.7 Kb upstream of promoter, D2S2187 in intron 1 at +43987 position +92031 A/T, +92344 C/T, +92817 C/T SNPs in intron 7, CA repeat at +99095 in the intron 11, +110832 A/G in splice variant exon 17 and +131237 C/T in intron 24 and the haplotypes generated using four loci, in Indian population. In the present study both case control and family based association study was carried out for these polymorphisms. Further, the invention clearly defines that the variants identified would be useful for any kind of population of any geographical origin.

The present invention has also identified the functional role of +110832 A/G polymorphism in splice variant exon 17 expressed in platelets. The extended exon contains potential PEST sequence from amino acid 584- 607 with PESTfind score of +7.49 (www.at.embnt.org/embnet/tools/bio/pestfind/). The +110832 A/G (rs2278206) polymorphism causes a threonine to alanine substitution at position 604 in protein sequence resulting in a poor PEST sequence (PESTfind score +4.95). Thus a A to G base substitution at this particular locus can make the INPP4 enzyme resistant to calpain proteases as shown by western blot experiments from human platelets.

The present invention also reports the identification of novel splice variants in asthmatic individuals. One of the splice variants has deleted exon 16 of 219 base pairs. This results in deletion of 73 amino acids from the encoded protein. This splice variant can result in protein with altered function.

The inventors have also shown the lower expression of INPP4A protein in the allergen - induced mouse model of asthma than in the saline treated normal mice and its restoration after treatment with anti-inflammatory drugs such as steroid.

Accordingly, the present invention provides genetic variants of the human *Inositol polyphosphate 4-phosphatase (INPP4A)* gene useful for prediction of asthma, said variants including:
(a) The gene variant of SEQ ID No. 1 having 1-230 contiguous nucleotides containing the group of CA dinucleotides of locus M1 present 44.7 kb upstream of gene start site.
(b) The gene variant of SEQ ID No. 2 having 1-400 contiguous nucleotides containing the GT dinucleotides at locus M2.
(c) The gene variant of SEQ ID No. 3 having 1-159 contiguous nucleotides containing the CA repeat polymorphism at nucleotide 229 of M3 locus.
(d) The gene variant of SEQ ID No. 4 having 1-1036 contiguous nucleotides containing the A/T polymorphism at nucleotide 75 of locus S1, the C/T polymorphism at nucleotide 388 of locus S2 and the C/T polymorphism at nucleotide 861 of locus S3.
(e) The gene variant of SEQ ID No. 5 having 1-961 contiguous nucleotides containing the G/A polymorphism at nucleotide 147 of S4 locus.
(f) The gene variant of SEQ ID No. 6 having 1-1707 contiguous nucleotides containing the C/T polymorphism at nucleotide 1221 of S5 locus.
(g) The splice variant of SEQ ID No. 24 having 1-817 contiguous nucleotides containing a splice variant region.

Another aspect of the invention relates to a method of detecting and predicting predisposition to asthma by screening for INPP4A haplotypes and splice variants and their expression in a subject, the said method comprising the steps of:
[a] isolating DNA from samples selected from whole blood, semen, saliva, tears, urine, fecal material, sweat, buccal, skin or hair;
[b] providing primers having SEQ ID Nos. 7-18;
[c] amplifying and sequencing genomic DNA stretches using primers of SEQ ID Nos. 7-18, wherein primers of SEQ ID No. 7, 9, 11, 13, 15, 17 are forward primers and primers of SEQ ID NO. 8, 10, 12, 14, 16, 18 are reverse primers by:
[d] amplifying and sequencing the DNA stretch of SEQ ID No. 1 of the INPP4A gene using the primer combination of SEQ ID Nos. 7 and 8, SEQ. ID No.1 containing sequence corresponding to the microsatellite repeat at the M1 locus D2S2311;
[e] amplifying and sequencing the DNA stretch of SEQ ID No. 2 of the INPP4A gene using the primer combination of SEQ ID Nos. 9 and 10, SEQ. ID No.2 containing sequence corresponding to the microsatellite repeat at the M2 locus D2S2187 at position +43987 intron 1;
[f] amplifying and sequencing the DNA stretch of SEQ ID No. 3 of the INPP4A gene using the primer combination of SEQ ID Nos. 11 and 12, SEQ. ID No. 3 containing sequence corresponding to the microsatellite repeat at the M3 locus +99095;
[g] amplifying and sequencing the DNA stretch of SEQ ID No. 4 of the INPP4A gene using the primer combination of SEQ ID Nos. 13 and 14, SEQ. ID No. 4 containing the single nucleotide polymorphism (SNP) sites S1 locus +92031A/T, S2 locus +92344 C/T and S3 locus +92817C/T;
[h] amplifying and sequencing the DNA stretch of SEQ ID No.5 of the INPP4A gene using the primer combination of SEQ ID Nos. 15 and 16, SEQ. ID. No. 5 containing the SNP site at the S4 locus at position +110832A/G in splice variant exon17;
[i] amplifying and sequencing the DNA stretch of SEQ ID No.6 of the INPP4A gene using the primer combination of SEQ ID Nos. 17 and 18, SEQ. ID No. 6 containing the SNP site at the S5 locus at position +131237 C/T, and
[j] validating and identifying the specific INPP4A gene variants computationally by comparison with the known wild type INPP4A gene sequences; and
[k] isolating RNA from whole blood samples; and
[l] isolating and identifying splice variants of SEQ ID No. 24 using the primer combination of SEQ ID Nos. 25 and 26.

Other and further aspects, features, and advantages of the present invention will be apparent from the following description of the presently preferred embodiments of the invention given for the purpose of disclosure.

In a further aspect of the invention pharmacogenetic markers are provided corresponding to gene variants of *INPP4A* gene for predicting and detecting humans susceptible to asthma: SEQ ID No.1 associated with D2S2311 locus, SEQ ID No. 2 associated with D2S2187 locus, SEQ ID No. 3 associated with +99095 CA repeat, SEQ ID No. 4 associated with +92031 A/T, +92344 C/T, +92817 C/T locus, SEQ ID No. 5 associated with +110832 A/G, SEQ ID No. 6 associated with +131237 C/T and SEQ ID No. 24 associated with splice variants.

The following associations have been identified by the inventors between variants of the INPP4A gene and asthma:
- the D2S2311 microsatellite allelic variant at the M1 locus is associated with susceptibility to asthma χ²=43.441128, DF= 9, pvalue < 0.0001 and its association is confirmed in family based studies (p= 0.0007).
- the D2S2311 microsatellite allele 402 was found to be a risk allele with OR-2.289, 95% C.I. [1.5443- 3.3929] and confirmed in family based studies with χ² =3.96, p = 0.0464.
- the D2S2311 microsatellite allele 400 was found to be a protective allele with Odds Ratio 0.575, 95% C.I. [0.4098-0.8068] and confirmed in family based studies with χ² =11.306, p = 0.0008.
- the CA repeat allelic variants at locus M3 have been found to be associated with susceptibility to asthma χ²= 11.467334, p=0.000600 and confirmed in family based studies (p=0.008).
- the CA repeat allelic variant 154 at locus M3 has a frequency of 81 % in patients.
- the CA repeat at locus M3 wherein allelic variant 152 has a frequency of 19 % in patients.
- the CA repeat allelic variant 154 has been found to be a risk allele in a case-control study with OR 1.734, 95% C.I. [1.249-2.407]; this observation was also confirmed in a family based study χ2 = 7.078, p value = 0.008.
- +92031 A/T polymorphism at the S1 locus wherein allele T is at the S1 locus has frequency of 19 % in patients.
- +92031 A/T polymorphism at the S1 locus wherein allele A is at the S1 locus has frequency of 81 % in patients.
- the +92031 A/T polymorphism at the S1 locus is associated with susceptibility to asthma with χ²=7.05, p=0.00791 as confirmed in a family based study χ2 = 9, p value = 0.0027.
- +110832 A/G (rs2278206), a functionally important nonsynonymous polymorphism at the S4 locus, when allele A was found to be over transmitted to affected offspring in a family based study (χ2 = 11.504, p = 0.0007); the +110832 A/G polymorphism causes a threonine to alanine substitution at position 604 resulting in a poor PEST sequence, making the protein more stable.
- +131237 C/T polymorphism at the S5 locus when allele T was found to be over transmitted to affected offspring in a family based study (χ2 = 4.545, p = 0.03).

An embodiment of the present invention relates to determination of a four-locus haplotype selected from the thirty-six novel four-locus haplotypes generated using Mol, S1, M3 and S4 loci in a case-control study. The said novel haplotypes are 396_T_154_G, 398_A_152_A, 400_T_152_A, 400_A_152_A, 406_T_152_A, 406_A_156_T, 412_A_154_A, 400_T_154_A, 402_T_152_A, 404_A_156_T, 410_A_154_A, 404_A_152_G, 406_A_152_A, 404_A_152_A, 406_T_152_G, 396_A_152_G, 400_A_154_G, 402_T_154_A, 402_A_152_A, 404_T_154_A, 400_T_154_G, 396_T_152_G, 404_T_152_A, 398_A_152_G, 386_A_154_A, 402_T_152_G, 398_T_152_G, 404_A_154_G, 408_A_154_A, 406_A_154_A, 398_A_154_A, 404_T_152_G, 400_A_154_A, 400_T_152_G, 402_A_154_A, 404_A_154_A.

Still another embodiment of the present invention relates to the confirmation of twenty-eight novel four-locus haplotypes in families namely, 396_T_154_G, 398_A_152_A, 400_T_152_A, 400_A_152_A, 406_T_152_A, 406_A_156_T, 412_A_154_A, 400_T_154_A, 402_T_152_A, 404_A_156_T, 410_A_154_A, 404_A_152_G, 406_A_152_A, 404_A_152_A, 406_T_152_G, 396_A_152_G, 400_A_154_G, 402_T_154_A, 402_A_152_A, 404_T_154_A, 400_T_154_G, 396_T_152_G, 404_T_152_A, 398_A_152_G, 386_A_154_A, 402_T_152_G, 398_T_152_G, 404_A_154_G, 408_A_154_A, 406_A_154_A, 398_A_154_A, 404_T_152_G, 400_A_154_A, 400_T_152_G, 402_A_154_A, 404_A_154_A

In yet another embodiment of the invention, the novel haplotype 402_A_154_A (loci M1_S1_M3 _S4) is detected as a risk factor for asthma. It was found to have percentage frequency 16.14 % in patients in case-control study.

The haplotype 402_A_154_A was strongly associated with occurrence of asthma (odds ratio 3.68 with 95% CI: 2.2977, 5.916, p value < 0.0001) indicating high risk in a case-control study. Moreover, the risk haplotype 402_A_154_A was also found to be over-transmitted to affected offspring in a family based study (χ2=4.2714, DF= 1, p value = 0.038).

The haplotype of loci M1_S1_M3 _S4 400_A_154_A was found to have percentage frequency of 7.29% in patients.

The haplotypes 400_T_152_G and 400_A_154_A were found to be negatively associated with occurrence of atopic asthma indicating protective haplotypes. Confirmation of negative association of the 400_T_152_G haplotype with occurrence of atopic asthma was obtained in a family-based study (χ²=8.065, DF= 1, pvalue = 0.0045).

Splice variants of the INPP4A gene have additionally been identified in atopic asthmatic subjects.

INPP4A protein expression is reduced in ovalbumin sensitized and challenged mice compared to saline treated controls; there is restoration after treatment with a known anti-inflammatory steroid agent, such as dexamethasone.

Yet another aspect of the invention relates to a diagnostic kit comprising pharmacogenetic markers having nucleotides of SEQ ID Nos. 1, 2, 3, 4, 5, 6 and 24 along with an instruction manual for detecting and predicting predisposition to asthma in a subject. Optional additional components of the kit include, for example, restriction enzymes, reverse-transcriptase or polymerase and the substrate.

### DETAILED METHODOLOGY

### Isolation of genomic DNA from peripheral blood leukocytes of the atopic asthmatic patients and the normal control individuals:

Genomic DNA was isolated from the peripheral blood of the patients and control individuals using a modified salting out procedure (Nagarkatti R et al., 2002). Briefly, 10ml blood was obtained from patients and unrelated control individuals using ACD Vaccutainers (BD Biosciences, San Jose, CA, USA). Equal volume of ice cold C1 buffer (4X) was added and then 30ml of ice cold sterile water was added to cause cell membrane lysis (Promega Genomic DNA Isolation Handbook). Following this, the nuclei were pelleted at 1300xg for 15 min at 4°C. The pellet was washed again with 1X C1 buffer. 12 ml of nuclear lysis buffer was added with 0.8ml of 10% SDS. 50µl of a 20µg/µl solution of proteinase-K was added and the pellet resuspended by brief vortexing. After incubation at 65°C for 2-3 hrs, the proteinaceous material was precipitated with the addition of 4 ml of 6M NaCl. After centrifugation for 15 min at 2500 rpm, the supernatant was transferred to another tube and two volumes of absolute ethanol (at room temperature) was used to precipitate the DNA (Miller et al., 1988). The precipitated DNA was then washed with 70% ethanol twice, air-dried, and dissolved in TE buffer. Appropriate dilutions (1:100, in T.E buffer) were used to determine the OD at 260nm and 280nm. DNA quality was assessed using the 260nm/280nm ratio. The stock solution of the DNA was diluted to 50ng/µl and used for PCR amplification and genotyping experiments. The stock DNA solution was stored at -20°C.

### Identification of putative repeats in and around the INPP4A gene using RepeatMasker^{™} Software:

Besides two known microsatellites, namely D2S2311, D2S2187, five repetitive sequences were identified in the *INPP4A* gene in the study population using RepeatMasker^{™} Software, but only CT repeat in intron 11 was found to be polymorphic and studied in detail (Figure 1B).

### Designing and synthesis of oligonucleotide primers for PCR amplification of INPP4A gene variants:

Primers were designed using DNASTAR Primer Select Software SEQ ID: 7, 8, 9, 10, 11, 12, 13, 14, 15, 16, 17, 18, 19, 20, 21, 22, 23, 25 and 26. The reverse primers for the repeat polymorphisms were labeled with 6- FAM for detection by fragment analysis on 3100 automated capillary array sequencer.

### PCR amplification conditions for different primer sets:

### PCR amplification of SEQ ID #1 and SEQ ID #2:

### Primers: SEQ ID # 7, 8 and SEQ ID # 9, 10

PCR amplification of genomic DNA samples isolated from peripheral blood leukocytes of the atopic asthmatic patients and normal control individuals using the above said primers in a pooled reaction. PCR was carried out in a total volume of 5µl containing 25ng of genomic DNA, 1.0 pmol each of 6-FAM-labeled reverse primers and non-labeled forward primers, 1.5Mm MgCl₂, 0.25mM of each dNTP, 0.03U/µl of Taq DNA polymerase (Bangalore Genie, India) and the buffer recommended by the supplier. After PCR, 1µl of the PCR product was loaded with and internal size standard (PET labeled) on ABI Prism 3100 Genetic Analyzer (Applied Biosystems). Fragment lengths were determined using the (Genotyper 3.7, Applied Biosystems).

### PCR conditions:

Denaturation at 94 degree C for 5 minutes, Thirty five cycles of denaturation at 94 degree C for 30 seconds, annealing at 65 degree C for 30 seconds, extension at 72 degree C for 30 seconds; followed by a 10 minutes 72 degree C segment extension period.

### PCR amplification of SEQ ID #3:

### Primer: SEQ ID # 11 and 12

PCR amplification of genomic DNA samples isolated from peripheral blood leukocytes of the atopic asthmatic patients and normal control individuals using the above said primers. PCR was carried out in a total volume of 5µl containing 25ng of genomic DNA, 1.0 pmol each of a 6-FAM-labeled forward primer and a non-labeled reverse primer, 1.5Mm MgCl₂, 0.25mM of each dNTP, 0.03U/µl of Taq DNA polymerase (Bangalore Genie, India) and the buffer recommended by the supplier. After PCR, 1µl of the PCR product was loaded with and internal size standard (PET labeled) on ABI Prism 3100 Genetic Analyzer (Applied Biosystems). Fragment lengths were determined using the (Genotyper 3.7, Applied Biosystems).

### PCR conditions:

Denaturation at 94 degree C for 5 minutes, Thirty five cycles of denaturation at 94 degree Cfor 30 seconds, annealing at 68 degree C for 30 seconds, extension at 72 degree C for 30 seconds; followed by a 10 minutes 72 degree C segment extension period.

### PCR amplification of SEQ ID #4:

### Primer: SEQ ID # 13 and 14

PCR amplification of genomic DNA samples isolated from peripheral blood leukocytes of the atopic asthmatic patients and normal control individuals using the above said primers. PCR was carried out in a total volume of 20 µl containing 50 ng of genomic DNA, 2.0 pmol each of a forward and reverse primer, 1.5Mm MgCl₂, 0.25mM of each dNTP, 0.03U/µl of Taq DNA polymerase (Bangalore Genie, India) and the buffer recommended by the supplier. Purified PCR product was used for Snapshot reaction.

### PCR conditions:

Denaturation at 94 degree C for 5 minutes, Thirty five cycles of denaturation at 94 degree C for 45 seconds, annealing at 66 degree C for 45 seconds, extension at 72 degree C for 45 seconds; followed by a 10 minutes 72 degree C segment extension period.

### PCR amplification of SEQ ID #5:

### Primer: SEQ ID # 15 and 16

PCR amplification of genomic DNA samples isolated from peripheral blood leukocytes of the atopic asthmatic patients and normal control individuals using the above said primers. PCR was carried out in a total volume of 20 µl containing 50 ng of genomic DNA, 2.0 pmol each of a forward and reverse primer, 1.5M MgCl₂, 0.25mM of each dNTP, 0.03U/µl of Taq DNA polymerase (Bangalore Genie, India) and the buffer recommended by the supplier. Purified PCR product was used for Snapshot reaction.

### PCR conditions:

Denaturation at 94 degree C for 5 minutes, Thirty five cycles of denaturation at 94 degree C for 45 seconds, annealing at 66 degree C for 45 seconds, extension at 72 degree C for 45 seconds; followed by a 10 minutes 72 degree C segment extension period.

### PCR amplification of SEQ ID # 6:

### Primer: SEQ ID # 17 and 18

PCR amplification of genomic DNA samples isolated from peripheral blood leukocytes of the atopic asthmatic patients and normal control individuals using the above said primers. PCR was carried out in a total volume of 20 µl containing 50 ng of genomic DNA, 2.0 pmol each of a forward and reverse primer, 1.5Mm MgCl₂, 0.25mM of each dNTP, 0.03U/µl of Taq DNA polymerase (Bangalore Genie, India) and the buffer recommended by the supplier. Purified PCR product was used for Snapshot reaction.

### PCR conditions:

Denaturation at 94 degree C for 5 minutes, Thirty five cycles of denaturation at 94 degree C for 45 seconds, annealing at 68 degree C for 45 seconds, extension at 72 degree C for 45 seconds; followed by a 10, minutes 72 degree C segment extension period.

### DIRECT SEQUENCING OF THE PURIFIED PCR PRODUCTS

Direct sequencing of the purified PCR products using dye terminator chemistry was carried out on an ABI Prism 3100 automated DNA sequencer for gene segments with SEQ ID # 4, 5 and 6. Sequencing was carried out using specific primers viz: 13, 14, 15, 16, 17 and 18 on an ABI 3100 capillary sequencer (Applied Biosystems, Foster City, CA, USA) for a minimum of 20 atopic asthmatic and 20 control individuals. PCR product was gel purified for sequencing. Briefly, sequencing primers, diluted to 1pmol per µl, and 75-150ng/µl PCR product were added to 5µl reaction mix, and volume made up to 10µl with autoclaved MilliQ water as per the Big Dye Terminator kit instructions (Applied Biosystems, Foster City, CA, USA). PCR was set up with the following conditions: 96 °C for 5 seconds, 55°C for 30 seconds and 60 °C for 4 minutes. Sequencing reactions were purified with 70% ethanol washes to remove unincorporated primers and fluorescent ddNTPs. Briefly, 26µl autoclaved MilliQ water was added to the sequencing reaction. Sixty-four microliters of chilled 100% ethanol was added to the tubes and vortexed. The tubes were centrifuged at 16,000 g for 20 minutes at room temperature. Washes were performed with 70% ethanol by centrifugation at 16,000 g for 5 minutes. The pellets were air dried and resuspended in 10µl of 100% Hi-Di formamide. The tubes were incubated at 94 °C for 5 minutes and placed in the 3100 Automated Sequencer. Sequence analysis was carried out using Sequence Navigator (ver 2.1, Applied Biosystems, Foster City, CA, USA) and DNAStar (ver 1.1, DNASTAR) software. Homozygous and heterozygous alleles were scored manually.

### Genotyping of +92031 A/T, +92344 C/T, +92817 C/T, +110832 A/G and +131237 C/T polymorphisms:

The +92031 A/T, +92344 C/T, +92817 C/T, +110832 A/G and +131237 C/T polymorphisms were selected on the basis of linkage disequilibrium among all the SNPs (Figure2) and studied using SNaPshot. ddNTP Primer Extension Kit (Applied Biosystems, Foster City, USA). SnaPshot PCR was carried out using 50ng purified PCR template, 1 pmol primer with SEQ Ids 19, 20, 21, 22 and 23 resp. and ABI ready reaction mix and 1X dilution buffer (as supplied by the manufacturer). PCR was set up with the following conditions: 96°C for 10 seconds, 58°C for 5 seconds and 60°C for 30 seconds for a total of 30 cycles. To clean up the primer extension reaction, 1U of calf intestinal phosphatase (CIP) diluted in 10X NEB3 (New England Biolabs), was added to the reaction mixture and the mixture was incubated at 37°C for 1 hour, followed by an incubation for 15 minutes at 72°C for enzyme inactivation. These samples were subsequently electrophoresed using the ABI Prism 3100 Genetic Analyzer as per the manufacturer's instructions. The results were analyzed using the program ABI Prism GeneScan^{™} and Genotyper^{™} (Applied Biosystems, Foster City, USA).

### Functional significance of +110832 A/G (rs2278206) polymorphism, in the extended exon 17 splice variant α 3:

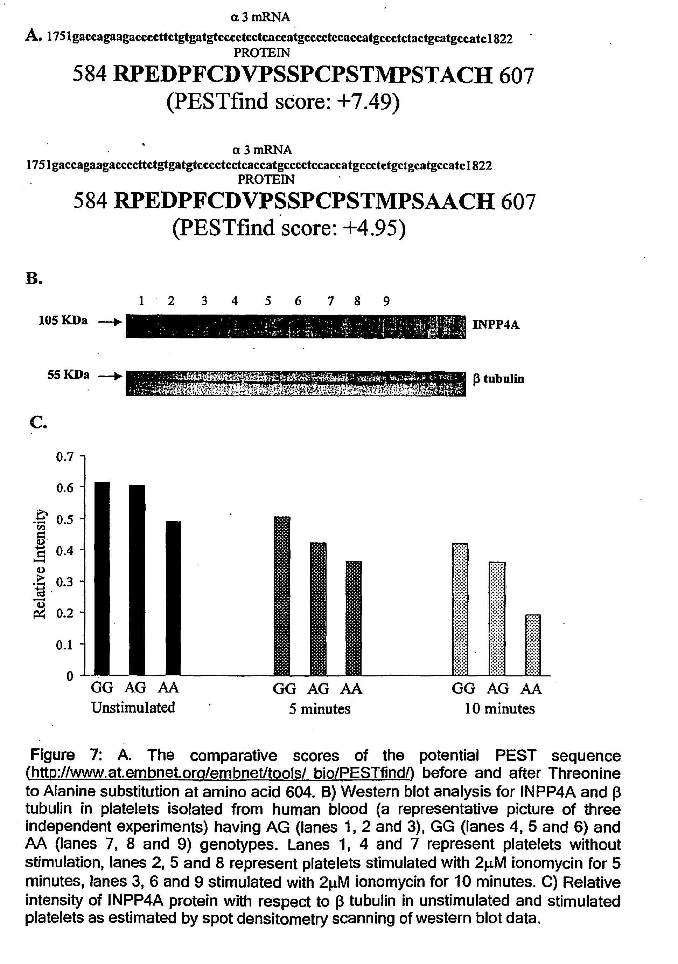

The PEST sequences in the splice variant α 3 were identified using PESTFIND tool of ExPASY Proteomic database (www.at.cmbnt.org/embnet/tools/bio/pestfind/). The protein sequence was downloaded from NCBI (AAK58870). The 977 amino acid splice form of protein contains additional amino acids from extended exon 17. The +110832 A to G polymorphism results in a nonsynonymous threonine to alanine substitution at position 604 in the amino acid sequence. The amino acid sequence was changed at 604 position from threonine to alanine and this sequence was again run through PESTFIND for finding PEST sequences. Platelets were isolated from peripheral blood of normal healthy individuals having AA, GG and AG genotypes for A+110832G polymorphism and stimulated with 2 µM ionomycin in platelet suspension buffer for 5 and 10 minutes, respectively. Western blot was performed using goat polyclonal antibody sc-12315 and anti-goat HRP antibody. Densitometry scanning of the bands was done using AlphaImager (Alpha Innotech Corporation, San Leandro, CA) (Figure 7).

### Identification of novel splice variants in the exon 15 - exon 19 region of INPP4A using primers with SEQ ID # 25 and 26:

RNA was isolated from the total leukocytes of 12 atopic asthmatics and 6 normal individuals using EZ-RNA isolation kit, following manufacturer's directions (Biological Industries). cDNA was made from 10 µg of total RNA using cDNA Archive kit from Applied Biosystems. PCR was carried out in a total volume of 20 µl containing cDNA corresponding to 100 ng of starting RNA, 2.0 pmol each of forward and reverse primer, 1.5Mm MgCl₂, 0.25mM of each dNTP, 0.03U/µl of Taq DNA polymerase (Bangalore Genie, India) and the buffer recommended by the supplier. PCR conditions:

Denaturation at 94 degree C for 5 minutes, Thirty cycles of denaturation at 94 degree C for 45 seconds, annealing at 65 degree C for 45 seconds, extension at 72 degree C for 45 seconds; followed by a 10 minutes 72 degree C segment extension period.

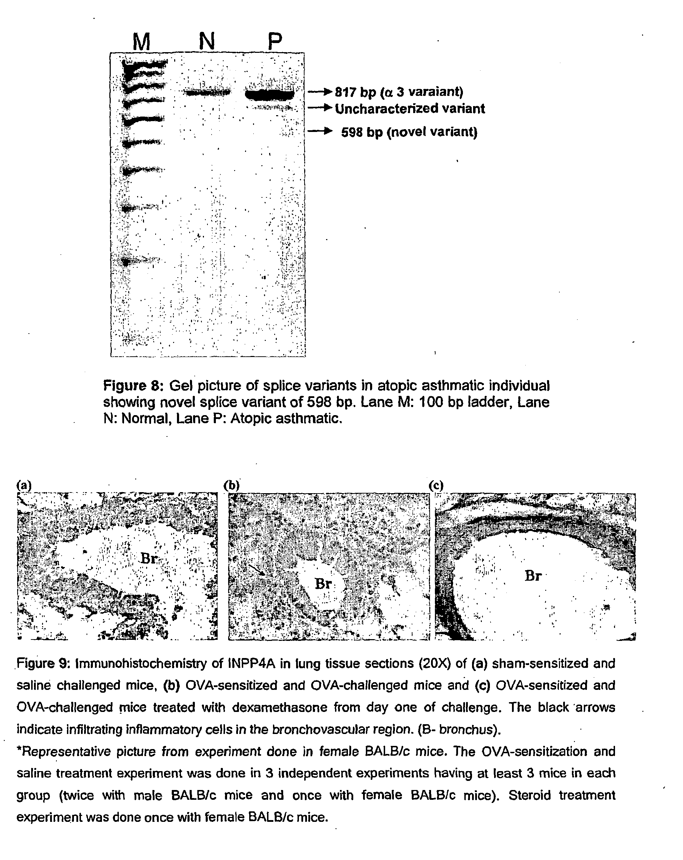

The 598 base pair splice variant (Figure 8) was sequenced as described above and the deletion of exon 16 was was confirmed.

### Calculating and estimating the frequency of repeat polymorphisms D2S2311, D2S2187 and that of +99095 CA, single nucleotide polymorphisms +92031 A/T, +92344 C/T, +92817 C/T, +110832 A/G and +131237 C/T:

**CLUMP** software with Monte Carlo simulations has been used to test the allelic association of for multi-allelic markers with disease phenotype for repeat polymorphisms. Odds ratios were calculated and Chi-square tests were performed to study the association with disease phenotype. The repeats have been denoted according to the fragment length where sizing was done using internal size standard during gene scan. SNPs are designated as S1, S2, S3, S4 and S5.

### Estimating the frequencies of haplotypes generated using four loci in the normal individuals and atopic asthmatic patients for finding association between these haplotypes and the disease:

Novel haplotypes for loci M1, S1, M3 and S4, have been generated using the PHASE program for the patient (N=192) and control (N= 272) groups (Stephens M, Am J Hum Genet. Nov 73:1162-9, 2003). Default parameters with 100 iterations were used to generate the haplotypes (http://archimedes.well.ox.ac.uk/pise/PHASE-simple.html, PHASE Ver. 2.0.2). Chi-squares and Odds ratios were calculated for association with phenotype.

### Transmission disequilibrium (TDT) analysis of repeat polymorphisms D2S2311, D2S2187 and that of +99095 CA single nucleotide polymorphisms +92031 A/T, +92344 C/T, +92817 C/T, +110832 A/G and +131237 C/T and their haplotypic analysis in nuclear families:

In the families, allele-wise TDT analysis was done using TDT-sTDT (http://genomics.med.upenn.edu/spielman/TDT.htm). Haplotypic transmission to affected individual was observed using TRANSMIT (Transmit, version 2.5.4).

So the matter in which the above-mentioned features, advantages and the objects of the invention, as well as others, which will become clear, are attained and can be understood in detail. These drawings form a part of the specification. It is to be noted, however, that the appended drawings illustrate preferred embodiments of the invention and therefore not to be considered limiting in their scope.

### Developing ovalbumin sensitized and challenged mouse model of asthma and saline treated control mice:

The BALB/c mice were sensitized by intra-peritoneal injections of 20 µg chicken egg ovalbumin (OVA) (grade V ≥ 98% pure, Sigma Chemical Co., St. Louis, MO, USA) in 0.2 ml saline adsorbed on 2 mg alum (sensitized) or alum alone (control) on days 0, 7 and 14. Mice were then challenged with aerosolized 3% ovalbumin (sensitized) or aerosolized saline (control) 30 minutes per day for 10 consecutive days (from day 22 to day 32). Responsiveness to methacholine (Sigma-Aldrich) was assessed in conscious, unrestrained mice by barometric plethysmography, using protocol, apparatus and software supplied by BUXCO (Troy, NY, USA). Mice were sacrificed 16 hrs after the last challenge and lung tissue were taken for immunohistochemistry.

### Treatment of ovalbumin induced mouse model of asthma with steroid:

The BALB/c mice were sensitized by intra-peritoneal injections of 20 µg chicken egg ovalbumin (OVA) (grade V ≥ 98% pure, Sigma Chemical Co., St. Louis, MO, USA) in 0.2 ml saline adsorbed on 2 mg alum on days 0, 7 and 14. Mice were then challenged with aerosolized 3% ovalbumin 30 minutes per day for 10 consecutive days (from day 22 to day 32). Treatment with 1 mg/kg dexamethasone was given starting from the day of last sensitization to last challenge (day 14-32). Bronchial hyperresponsiveness was measured as described previously using BUXCO plethysmograph (Troy, NY, USA).

Mice were sacrificed 16 hrs after the last OVA challenge and lungs were subjected to immunohistochemistry.

### Immunohistochemistry of mouse lung tissue section for INPP4A protein:

Immunohistochemistry of mouse lung section was done using commercial goat polyclonal antibody sc-12315 (Santa Cruz Biotechnology, Inc.; CA, USA), raised against a peptide near the amino terminus of INPP4A of human and mouse origin following standard protocol with slight modifications (Ather MH et al, 2004). Goat gamma globulin was used as isotype control (Jackson Immunoresearch Laboratories, Inc.; PA, USA).

### Brief description of the accompanying drawings:

**Figure 1A** shows.the schematic presentation of the different known spliceofonms of INPP4A, **Figure 1B** shows a schematic presentation of the different polymorphic regions of the *INPP4A* gene studied. The D2S2311 repeat, 44.7 kb upstream of the promoter, is denoted as M1. The D2S2187 repeat is referred to as M2, the CA repeat polymorphism at +99095 is denoted as M3. All five of the polymorphisms are also shown in sequence context below the gene.
**Figure 2** shows graphical overview of linkage disequilibrium for the eight loci studied using GOLD.
**Figure 3** shows the distribution of D2S2311 alleles in patient and control group. The figure depicts the allele frequencies at the D2S2311 repeat locus with the fragment sizes depicted on the X-axis and their respective frequencies on the Y-axis.
**Figure 4** shows allelic and genotypic distribution of +92031 A/T (S1) in case-control study.
**Figure 5** shows allelic and genotypic distribution of CA repeat polymorphism in intron 11 in case- control study.
**Figure 6** shows the distribution of haplotypes (M1_S1_M3_S4) in Patients and Controls (Np= 192, Nc= 272) (Table 2). The figure depicts the haplotype frequencies on the X-axis and their respective frequencies on the Y-axis.
**Figure 7** shows the potential PEST sequence and its experimental validation in the extended exon 17 splice variant region and comparison with Thr to Ala substitution variant.
**Figure 8** shows the gel picture of known α 3 splice variant along with the novel splice variants identified in atopic asthmatic patients.
**Figure 9** shows the immunohistochemistry of mouse lung sections from saline treated control mice, ovalbumin sensitized mice and the steroid treated ovalbumin sensitized and challenged mice.

### Association of the repeat loci with atopic disorders such as asthma

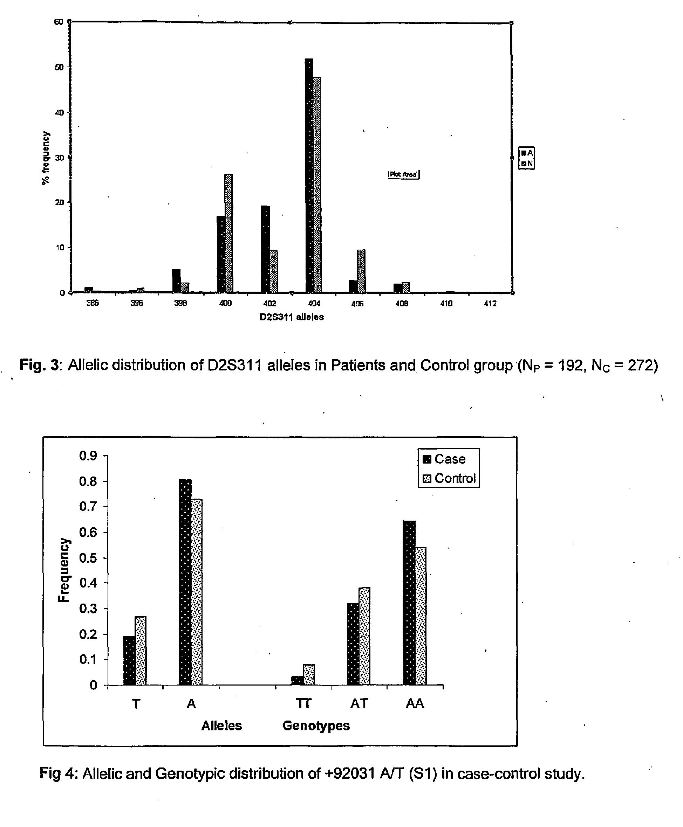

To demonstrate the association of the M1 and M2 and M3 repeat locus with atopic disorders such as asthma, chi square test was performed using CLUMP software. CLUMP is a program designed to assess the significance of the departure of observed values in a contingency table from the expected values conditional on the marginal totals. The significance is assessed using a Monte Carlo approach, by performing repeated simulations to generate tables having the same marginal totals as the one under consideration, and counting the number of times that a chi-squared value associated with the real table is achieved by the randomly simulated data. This means that the significance levels assigned should be unbiased (with accuracy dependent on the number of simulations performed) and that no special account needs to be taken of continuity corrections or small expected values. The method is described in full in: Sham PC & Curtis D.1995. Monte Carlo tests for associations between disease and alleles at highly polymorphic loci. Ann Hum Genet. 59: 97-105. A significantly different pattern of distribution of the alleles between the two groups was obtained; for alleles at M1 locus and found to be associated with susceptibility to asthma χ2=43.441128, DF= 9, p value < 0.0001. Allele 402 was found to be a risk allele with Odds Ratio 2.289, 95% C.I. [1.5443- 3.3929] allele 400 was found to be a protective allele with Odds Ratio 0.575, 95% C.I. [0.4098-0.8068] as calculated by 2x2 table (http://home.clara.net/sisa/twoby2.htm) (Figure 3). Similar results were obtained in family based association studies (p value = 0.0007) (Table 3a).

There was no significantly different pattern of distribution of the alleles between the two groups for M2 (p=0.214179) in case-controls (Table 3b).

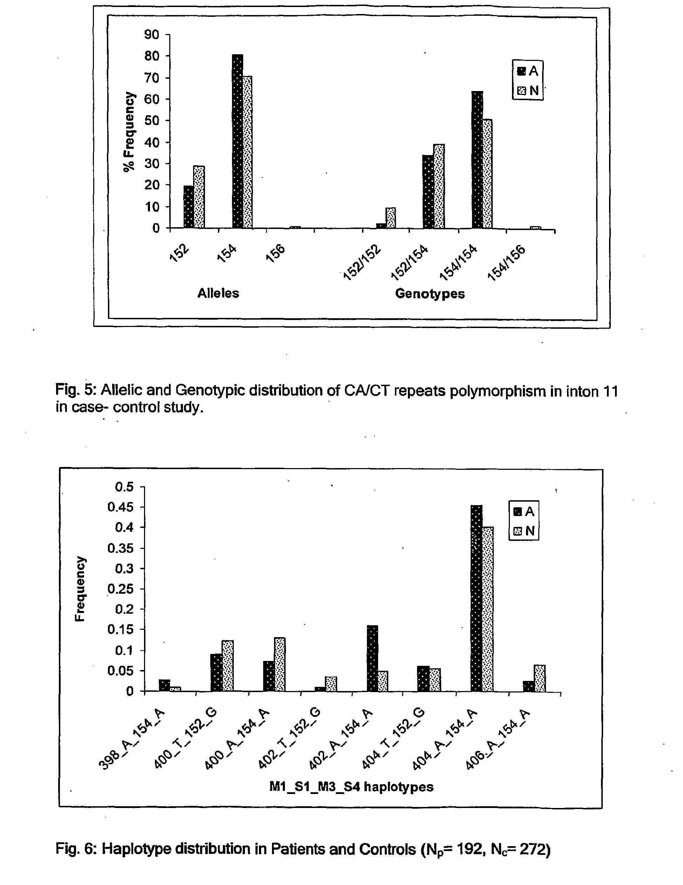

The locus M3 was also found to be significantly associated with asthma (p=0.0006). The CA repeat allelic variants at locus M3 have been found to be associated with susceptibility to asthma χ²= 11.467334, p=0.0006. The CA repeat allelic variant 154 has been found to be a risk allele in case-control study with OR 1.734, 95% C.I. [1.249-2.407] (Figure 5). This observation was also confirmed in family based study χ2 = 7.078, p value = 0.008 (Table 3f).

### Demonstration of association of +92031 A/T (S1) polymorphism with asthma:

To demonstrate the association of +92031 A/T polymorphism, Armitage trend test was performed. The pattern of distribution of the three genotypes, AA, AT and TT was significantly different in the two groups studied (χ2=7.05, p=0.008). The A allele was predominant in the cases as compared to the control group {OR=1.542, 95% C.I. (1.121-2.120), p = 0.0075} (Figure 4). Similarly, the T allele was found to be over transmitted to affected offsprings in family based study using TDT-sTDT program (χ2=9, p=0.0027) (Table 3c).

### Demonstration of association of +110832 A/G (S4) polymorphism with asthma:

To demonstrate the association of +110832 A/G nonsynonymous polymorphism, Armitage trend test was performed in case- control study. The risk allele T showed a marginal association with the disease phenotype {Odds_ratio=1.324, 95% C.I. (0.964-1.819), χ2=3.01, p=0.08). The A allele was found to be over transmitted to affected offsprings in family based study using TDT-sTDT program (χ2=11.5, p=0.0007) (Table 3g).

### Generation of haplotypes:

We then used the PHASE program to generate haplotypes for the patient and control groups. The program PHASE implements a new statistical method for reconstructing haplotypes from population genotype data. Experiments with the software on both real and simulated data indicate that it can provide an improvement on the EM algorithm for reconstructing haplotypes. It allows for missing genotype data and also can handle more than one locus irrespective of the polymorphism, for example SNPs and repeats can be analyzed simultaneously. Based on the output from the software the probability values of the haplotypes are also predicted and can be utilized to differentiate more confident haplotypes. The PHASE software is suitable for genetic distances of 100 cM or less. Similary family based haplotypic analysis was performed using TRANSMIT program. TRANSMIT tests for association between genetic marker and disease by examining the transmission of markers from parents to affected offspring. The tests are based on a score vector which is averaged over all possible configurations of parental haplotypes and transmissions consistent with the observed data.

In case-control study, the haplotypes whose expected frequency was larger than 0.025, in either of the two groups are shown in Table 2 (Figure 6). The odds in favor of patients rather than controls having 402_A_154_A haplotype were 3.68 with 95% CI: 2.2977, 5.916. The corresponding likelihood ratio χ2 tests showed p-value less than 10-5. TRANSMIT also showed similar results in family based association study (χ2=4.2714, DF= 1, p value = 0.038). Thus the 4-locus haplotype, 402_A_154_A was strongly associated with asthma. On the other hand, the odds in favor of patients rather than controls having haplotype 400_T_152_G and 400_A_154_A were 0.16, 95% CI: 0.46-1.09 and 0.12, 95% CI: 0.33-0.82 respectively. The haplotype 400_T_152_G was also found to be negatively associated with occurrence of atopic asthma in family based study. (χ²=8.065, DF= 1, p value = 0.0045). Thus, haplotypes 402_A_154_A and 400_T_152_G were identified to be major risk and protective haplotypes respectively.

### Functional significance of +110832 A/G (rs2278206) polymorphism in the extended exon 17 splice variant α 3:

Threonine to Alanine substitution at amino acid 604 in the α 3 splice variant of INPP4A gene resulted in a change in the PESTfind score from +7.49 to +4.95 making it a poor PEST sequence. Protein from platelets of individuals with AA genotype was found to be more susceptible to degradation as seen by nearly 60% degradation within 10 min of stimulation whereas the protein from individuals with GG genotype was less susceptible (nearly 31 % degradation). Also, the basal levels of INPP4A protein from individuals with AA genotypes was found to be lower (data not shown), suggesting poor stability in vivo as well (Figure 7).

### Identification of novel splice variants in the exon 15 - exon 19 region of INPP4A:

Novel splice variant of 598 base pair was identified in asthmatic patients. This variant has deleted exon 16 (219 bases) as confirmed by sequencing (Figure 8).

### Significant decrease in the INPP4A protein expression in allergen-induced mouse model of asthma:

The INPP4A protein was found to be expressed at significantly lower levels in ovalbumin induced mouse model of asthma as compared to saline treated controls (Figure 9).

### Restoration of INPP4A protein in ovalbumin induced mice by treatment with anti-inflammatory agent:

The levels of INPP4A protein were found to be restored in case of steroid treated ovalbumin induced mouse model of asthma. Noticeably, the expression of INPP4A protein after steroid treatment was found to be even higher than the saline treated control mice (Figure 9).

### Analysis of Polymorphisms:

### A. Preparation of Samples:

Polymorphisms are detected in a target nucleic acid from an individual being analyzed. For assay of genomic DNA, virtually any biological sample (other than pure red blood cells) is suitable. For example, convenient tissue samples include whole blood, semen, saliva, tears, urine, fecal material, sweat, buccal, skin and hair. For assay of cDNA or mRNA, the tissue sample must be obtained from an organ in which the target nucleic acid is expressed.

Many of the methods described below require amplification of DNA from target samples. This can be accomplished by e.g., PCR. See generally PCR Technology: Principles and Applications for DNA Amplification (ed. H. A. Erlich, Freeman Press, N.Y., N.Y., 1992); PCR Protocols: A Guide to Methods and Applications (eds. Innis, et al., Academic Press, San Diego, Calif.,1990); Mattila et al., Nucleic Acids Res. 19, 4967 (1991) and U.S. Pat. No. 4,683,202 (each of which is incorporated by reference for all purposes).

Other suitable amplification methods include the Ligase Chain Reaction (LCR) (see Barringer KJ et al, Gene 89:117-22, 1990; Friedhoff P et al, Anal Biochem 215:9-16, 1993) and Nucleic Acid Based Sequence Amplification (NASBA). The latter two amplification methods involve isothermal reactions based on isothermal transcription, which produce both single stranded RNA (ssRNA) and double stranded DNA (dsDNA) as the amplification products in a ratio of about 30 or 100 to 1, respectively.

### B. Detection of Polymorphisms in Target DNA:

There are two distinct types of analysis depending on whether a polymorphism in question has already been characterized or not. The first type of analysis is sometimes referred to as de novo characterization. This analysis compares target sequences in different individuals to identify points of variation, i.e., polymorphic sites. By analyzing groups of individuals representing the greatest ethnic diversity among humans and greatest breed and species variety in plants and animals, patterns characteristic of the most common alleles/haplotypes of the locus can be identified, and the frequencies of such populations in the population determined, Additional allelic frequencies can be determined for subpopulations characterized by criteria such as geography, race, or gender. The de novo identification of the polymorphisms of the invention is described in the Examples section. The second type of analysis is determining which form(s) of a characterized polymorphism are present in individuals under test. There are a variety of suitable procedures, which are discussed in turn.

### 1. Repeat detection (size variation detection):

The design and use of primers flanking the sequence contain the repeat sequence or other polymorphic elements, which lead to a size difference. PCR amplification of the sequence leads to the presence of a pool of amplified products that differ by the specific repeat or polymorphism size. These size differences can then be detected using gel based, charge based methods. Usually for gel-based detection one of the primers is labeled with a fluorescent compound which can then be excited and detected using a CCD camera or other methods.

### 2. Allele-Specific Probes:

The design and use of allele-specific probes for analyzing polymorphisms is described by e.g., Saiki et al., Nature 324, 163-166, 1986; Dattagupta, EP 235,726, Saiki, WO 89/11548. Allele-specific probes can be designed that hybridize to a segment of target DNA from one individual but do not hybridize to the corresponding segment from another individual due to the presence of different polymorphic forms in the respective segments from the two individuals.

### 3. Allele-Specific Primers:

An allele-specific primer hybridizes to a site on target DNA overlapping a polymorphism and only primes amplification of an allelic form to which the primer exhibits perfect complementarity. This primer is used in conjunction with a second primer which hybridizes at a distal site. See, e.g., WO 93/22456.

### 4. Direct-Sequencing:

The direct analysis of the sequence of polymorphisms of the present invention can be accomplished using either the dideoxy chain termination method or the Maxam Gilbert method (see Sambrook et al., Molecular Cloning, A Laboratory Manual (2nd Ed., CSHP, New York 1989); Zyskind et al., Recombinant DNA Laboratory Manual, (Acad. Press, 1988)).

### 5. Denaturing Gradient Gel Electrophoresis:

Amplification products generated using the polymerase chain reaction can be analyzed by the use of denaturing gradient gel electrophoresis. Different alleles can be identified based on the different sequence-dependent melting properties and electrophoretic migration of DNA in solution. Erlich, ed., PCR Technology, Principles and Applications for DNA Amplification, (W.H. Freeman and Co, New York, 1992), Chapter 7.

### 6. Single-Strand Conformation Polymorphism Analysis:

Alleles of target sequences can be differentiated using single-strand conformation polymorphism analysis, which identifies base differences by alteration in electrophoretic migration of single stranded PCR products, as described in Orita et al., Proc. Nat. Acad. Sci. 86, 2766-2770, 1989. Amplified PCR products can be generated as described above, and heated or otherwise denatured, to form single stranded amplification products. Single-stranded nucleic acids may refold or form secondary structures which are partially dependent on the base sequence. The different electrophoretic mobilities of single-stranded amplification products can be related to base-sequence difference between alleles of target sequences.

### Methods of Use:

After determining polymorphic form(s) present in an individual at one or more polymorphic sites, this information can be used in a number of methods.

### A. Correlation of Polymorphisms with Phenotypic Traits:

Atopic diseases are heterogeneous in nature and as such there are many sub-phenotypes and traits to which the association can be observed. The polymorphisms of the invention may contribute to the phenotype of an organism in different ways. As described above, the polymorphisms may act at various levels of cellular organization by which the disease phenotypes are observed as the end result. These polymorphisms may yield different selection advantages or disadvantages. For example, a heterozygous sickle cell mutation confers resistance to malaria, but a homozygous sickle cell mutation is usually lethal. A single polymorphism may affect more than one phenotypic trait.

Likewise, a single phenotypic trait may be affected by polymorphisms in different genes. Further, some polymorphisms predispose an individual to a distinct mutation that is causally related to a certain phenotype. Phenotypic traits include diseases that have known but hitherto unmapped genetic components. Phenotypic traits also include symptoms of, or susceptibility to, multifactorial diseases of which a component is or may be genetic, such as atopy, autoimmune diseases, inflammation, cancer, diseases of the nervous system, and infection by pathogenic microorganisms. Some examples of autoimmune diseases include systemic lupus erythematosus, rheumatoid arthritis, diabetes, multiple sclerosis, (insulin-dependent and non-independent), and Graves disease. Some examples of cancers include cancers of the breast, bladder, colon, brain, etc. As such, phenotypic traits also include characteristics, for example, susceptibility or receptivity to particular drugs or therapeutic treatments.

To perform association analysis of the disease phenotypes and genetic markers, the presence or absence of a set of polymorphisms (i.e. a polymorphic set) is determined for a set/ population of the individuals, some of whom exhibit a particular trait termed variously as case/ patients/ affected/ diseased individuals etc, and some of which exhibit lack of the trait termed variously as control individuals/ normal etc. The alleles of each polymorphism of the set are then counted to determine if the presence or absence of a particular allele or a set of alleles or a haplotype is associated with the trait of interest. Test for such associations can be performed by standard statistical methods such as a χ2 test etc. Based on the values obtained for the hypothesis tested for example, if the allele X is present more in patients than in controls and if the allele X is not present more in patients than in controls, the significance value is obtained. If this value lies in a particular range then it determines the significance level of the correlations. For example, it might be found that the presence of allele A1 at polymorphic site 1 correlates with cystic fibrosis disease. As a further example, it might be found that the combined presence of allele A1 at polymorphic site 1 and allele B1 at polymorphic site 2 correlates with 10 fold-increased severity of cystic fibrosis.

Such associations can be of immediate benefit if an extremely strong correlation exists. For example, detection of cystic fibrosis polymorphism A1 and B1 in a patient may allow for rapid diagnosis and discrimination from other diseases which exhibit similar phenotypes; it can also allow for treatment if available; it can allow for screening of neonates for detection and/or for susceptibility and/or risk assessment; it can allow for selection of better and improved management methods for the disease from those which are available; it may allow for the treatment to be given if it is determined that the polymorphic site also correlates with particular therapeutic regimes and that such therapeutic drugs are more beneficial to the patient than other drugs.

### B. Genetic Mapping of Phenotypic Traits:

The previous section concerns identifying correlations between phenotypic traits and polymorphisms that directly or indirectly contribute to those traits. The present section describes identification of a physical linkage between a genetic locus associated with a trait of interest and polymorphic markers that are not associated with the trait, but are in physical proximity with the genetic locus responsible for the trait and co-segregate with it. Such analysis is useful for mapping a genetic locus associated with a phenotypic trait to a chromosomal position, and thereby cloning gene(s) responsible for the trait. Please see (Altshuler D et al, 1998, N Engl J Med 338:1626; Cargill M et al, 1999, Nat Genet 22:231-8; Chang C, 1988, Proc Natl Acad Sci U S A 85:6856-60; Hacia JG et al, 1999, Nat Genet 22:164-7; Hirschhom JN et al, 2000, Proc Natl Acad Sci U S A 97:12164-9; Lander ES and Botstein D, 1986, Proc Natl Acad Sci U S A 83:7353-7; Lander ES, 1993, Nat Genet 4:5-6; Reich DE et al, 2001, Nature 411:199-204; Sachidanandam R et al, 2001, Nature 409:928-33. Genes localized by linkage can be cloned by a process known as directional cloning.

Computer programs are available for the calculation of lod scores for differing values of theta. Other references on linkage and disease mapping use above mentioned approaches include, Kreutz R et al, 1995, Proc Natl Acad Sci U S A 92:8778-82; de Gouyon B et al, 1993, Proc Natl Acad Sci U S A 90:1877-81; Julier C et al, 1990, Proc Natl Acad Sci U S A 87:4585-9; Oberle I et al, 1986, Proc Natl Acad Sci U S A 83:1016-20; Lathrop GM et al, 1984, Proc Natl Acad Sci U S A 81:3443-6; Cohen D et al, 1984, Proc Natl Acad Sci U S A 81:1774-8.

The same substrate can be used as a template for allele-specific oligonucleotide probes for detecting all of the polymorphisms listed. For initial screening purposes, the D2S2311 repeat polymorphism found 44.7 kb upstream of the human *INPP4A* gene could be useful as the allele 400 of this polymorphism is negatively associated, whereas the allele 402 is positively associated with asthma. For this locus, PCR was carried out in a total volume of 5µl containing 25ng of genomic DNA, 1.25 pmol each of a 6-FAM-labelled forward primer and a non-labeled reverse primer, 1.5Mm MgCl₂, 0.25mM of each dNTP, 0.03U/µl of Taq DNA polymerase (Bangalore Genie, India) and the buffer recommended by the supplier. After PCR, 1µl of the PCR product was loaded with an internal size standard (PET labeled) on ABI Prism 3100 Genetic Analyzer (Applied Biosystems). Fragment lengths were determined using the Genotyper Software version 3.7 (Applied Biosystems). If subsequently required, genotyping at the other seven loci, namely, +92031 A/T, +92344 C/T, +92817 C/T, +110832 A/G, +131237 C/T and +99095 CA repeat could also be carried out.

Genotyping the +92031 A/T, +92344 CT, +92817 C/T, +110832 A/G, +131237 C/T polymorphisms may be carried out using SnapShot primers of SEQ ID Nos. 19, 20, 21, 22, 23 respectively.

Additionally, oligonucleotide pairs may be employed for detecting novel splice variants of the INPP4A mRNA as indicated above. Optional additional components of a kit include, for example, restriction enzymes, reverse-transcriptase or polymerase, the substrate nucleoside triphosphates, means used to label (for example, an avidin-enzyme conjugate and enzyme substrate and chromogen if the label is biotin), and the appropriate buffers for reverse transcription, PCR, or hybridization reactions. Usually, the kit also contains instructions for carrying out the methods.

The following examples are given by way of illustration only and therefore should not be construed to limit the scope of the present invention.

### EXAMPLE 1

### Association of D2S2311 repeat locus with atopic disorders such as asthma:

The 401 bp DNA stretch of SEQ ID No. 1 of INPP4A gene having the D2S2311 repeat polymorphism was PCR amplified using novel primers of SEQ ID No. 7 and 8.PCR amplification of genomic DNA samples isolated from peripheral blood leukocytes of the atopic asthmatic patients and normal control individuals was done using the above said primers in a pooled reaction. PCR was carried out in a total volume of 5µl containing 25ng of genomic DNA, 1.0 pmol each of 6-FAM-labeled reverse primers and non-labeled forward primers, 1.5Mm MgCl2, 0.25mM of each dNTP, 0.03U/µl of Taq DNA polymerase (Bangalore Genie, India) and the buffer recommended by the supplier. After PCR, 1µl of the PCR product was loaded with and internal size standard (PET labeled) on ABI Prism 3100 Genetic Analyzer (Applied Biosystems). Fragment lengths were determined using the (Genotyper 3.7, Applied Biosystems). PCR was set up with the following conditions: Denaturation at 94 degree C for 5 minutes, Thirty five cycles of denaturation at 94 degree C for 30 seconds, annealing at 65 degree C for 30 seconds, extension at 72 degree C for 30 seconds; followed by a 10 minutes 72 degree C segment extension period.To demonstrate the association of the D2S2311 repeat locus with atopic disorders such as asthma, CLUMP software analysis was used. CLUMP is a program designed to assess the significance of the departure of observed values in a contingency table from the expected values conditional on the marginal totals. The significance is assessed using a Monte Carlo approach, by performing repeated simulations to generate tables having the same marginal totals as the one under consideration, and counting the number of times that a chi-squared value associated with the real table is achieved by the randomly simulated data. This means that the significance levels assigned should be unbiased (with accuracy dependent on the number of simulations performed) and that no special account needs to be taken of continuity corrections or small expected values. This analysis showed significant differences between the allele count distribution of patient and control groups (χ2=43.441128, DF= 9, pvalue < 0.0001). We observed a significantly different pattern of distribution of the alleles between the two groups; alleles 402 and 404 were over-represented in the patient group whereas alleles 400 and 406 were the major alleles in the control group. Allele 402 was found to be a risk allele with Odds Ratio 2.289, 95% C.I. [1.5443- 3.3929] allele 400 was found to be a protective allele with Odds Ratio 0.575, 95% C.I. [0.4098-0.8068] as calculated by 2x2 table (http://home.clara.net/sisa/twoby2.htm) (Figure 3).. Similar results were obtained in family based study (p value = 0.0007) (Table 3a).

### EXAMPLE 2

### Association of +92031 A/T polymorphism with atopic disorders such as asthma:

The 1036 bp DNA stretch of SEQ ID No. 4 of INPP4A gene having the +92031 A/T polymorphism was PCR amplified using novel primers of SEQ ID No. 13 and 14. The genotyping was done using SNaPshot. ddNTP Primer Extension Kit (Applied Biosystems, Foster City, USA). SnaPshot PCR was carried out using 50ng purified PCR template, 1 pmol primer with SEQ ID 19 and ABI ready reaction mix and 1X dilution buffer (as supplied by the manufacturer). PCR was set up with the following conditions: 96°C for 10 seconds, 58°C for 5 seconds and 60°C for 30 seconds for a total of 30 cycles. To clean up the primer extension reaction, 1U of calf intestinal phosphatase (CIP) diluted in 10X NEB3 (New England Biolabs), was added to the reaction mixture and the mixture was incubated at 37°C for 1 hour, followed by an incubation for 15 minutes at 72°C for enzyme inactivation. These samples were subsequently electrophoresed using the ABI Prism 3100 Genetic Analyzer as per the manufacturer's instructions. The results were analyzed using the program ABI Prism GeneScan^{™} and Genotyper^{™} (Applied Biosystems, Foster City, USA).To demonstrate the association of +92031 A/T polymorphism, Armitage trend test was performed. The pattern of distribution of the three genotypes, AA, AT and TT was significantly different in the two groups studied (χ2=7.05, p=0.008). The A allele was predominant in the cases as compared to the control group {OR=1.542, 95% C.I. (1.121-2.120), p = 0.0075} (Table1, Figure 4). Similarly, the A allele was found to be over transmitted to affected offsprings in family based study using TDT-sTDT program (χ2=9, p=0.0027) (Table 3c).

### EXAMPLE 3

### Association of +110832 A/G polymorphism with atopic disorders such as asthma:

The 961 bp DNA stretch of SEQ ID No. 5 of INPP4A gene having the +110832 A/G polymorphism was PCR amplified using novel primers of SEQ ID No. 15 and 16. The genotyping was done using SNaPshot. ddNTP Primer Extension Kit (Applied Biosystems, Foster City, USA). SnaPshot PCR was carried out using 50ng purified PCR template, 1 pmol primer with SEQ ID 22 and ABI ready reaction mix and 1X dilution buffer (as supplied by the manufacturer). PCR was set up with the following conditions: 96°C for 10 seconds, 58°C for 5 seconds and 60°C for 30 seconds for a total of 30 cycles. To clean up the primer extension reaction, 1U of calf intestinal phosphatase (CIP) diluted in 10X NEB3 (New England Biolabs), was added to the reaction mixture and the mixture was incubated at 37°C for 1 hour, followed by an incubation for 15 minutes at 72°C for enzyme inactivation. These samples were subsequently electrophoresed using the ABI Prism 3100 Genetic Analyzer as per the manufacturer's instructions. The results were analyzed using the program ABI Prism GeneScanTM and GenotyperTM (Applied Biosystems, Foster City, USA). To demonstrate the association of +110832 A/G polymorphism, Armitage trend test was performed in case-control study. The risk allele A showed a marginal association with the disease phenotype {Odds_ratio=1.324, 95% C.I. (0.964-1.819), χ2=3.01, p=0.08) (Table1). The A allele was found to be over transmitted to affected offsprings in family based study using TDT-sTDT program (χ2=11.5, p=0.0007) (Table 3e).

### EXAMPLE 4

### Association of INPP4A M1_S1_M3 _S4 locus haplotype with atopic disorders such as asthma:

To demonstrate haplotypic association PHASE program was used to generate haplotypes for the patient and control groups. The program PHASE implements a new statistical method for reconstructing haplotypes from population genotype data. Experiments with the software on both real and simulated data indicate that it can provide an improvement on the EM algorithm for reconstructing haplotypes. It allows for missing genotype data and also can handle more than one locus irrespective of the polymorphism, for example SNPs and repeats can be analyzed simultaneously. Based on the output from the software the probability values of the haplotypes are also predicted and can be utilized to differentiate more confident haplotypes. The PHASE software is suitable for genetic distances of 100 cM or less. Similarly, family based haplotypic analysis was performed using TRANSMIT program. TRANSMIT tests for association between genetic marker and disease by examining the transmission of markers from parents to affected offspring. The tests are based on a score vector which is averaged over all possible configurations of parental haplotypes and transmissions consistent with the observed data.

In case-control study, the haplotypes whose expected frequency was larger than 0.025, in either of the two groups are shown in Table 2 (Figure 6). The odds in favor of patients rather than controls having 402_A_154_A haplotype was 3.68 with 95% CI: 2.2977, 5.916. The corresponding likelihood ratio χ2 tests showed p-value less than 10-5. TRANSMIT also showed similar results in family based association study (χ2=4.2714, DF= 1, p value = 0.038).Thus the 4-locus haplotype, comprising loci M1_ S1_M3_S4, 402_A_154_A was strongly associated with asthma. On the other hand, the odds in favor of patients rather than controls having haplotype 400_T_152_G and 400_A_154_A were 0.16, 95% CI: 0.46-1.09 and 0.12, 95% CI: 0.33-0.82 respectively. The haplotype 400_T_152_G was also found to be negatively associated with occurrence of atopic asthma in family based study. (χ²=8.065, DF= 1, p value = 0.0045). Thus, haplotypes 402_A_154_A and 400_T_152_G were identified to be major risk and protective haplotypes respectively.

### EXAMPLE 5

### PEST analysis of +110832 A/G polymorphism:

To demonstrate the importance of Threonine to Alanine substitution at amino acid 604 in the α 3 splice variant of INPP4A gene. The extended exon contains potential PEST sequence from amino acid 584- 607 with PESTfind score of +7.49 (www.at.embnt.org/embnet/tools/bio/pestfind/). The +110832 A/G (rs2278206) polymorphism causes a threonine to alanine substitution at position 604 in protein sequence resulting in a poor PEST sequence (PESTfind score +4.95). Thus, Threonine to Alanine substitution at this particular locus can make the INPP4 enzyme resistant to calpain proteases (Figure 7 A).

### EXAMPLE 6

### Functional validation of +110832 A/G polymorphism:

To substantiate this hypothesis experimentally, platelets from normal healthy individuals of different genotypes at A+110832G (S4) were stimulated with ionomycin and the degradation of INPP4A was checked by western blot analysis. Protein from platelets of individuals with AA genotype was found to be more susceptible to degradation as seen by nearly 60% degradation within 10 min of stimulation whereas the protein from individuals with GG genotype was less susceptible (nearly 31% degradation). Also, the basal levels of INPP4A protein from individuals with AA genotypes was found to be lower (data not shown), suggesting poor stability in vivo as well (Figure 7b, 7c).

### EXAMPLE 7

### Novel splice variants of INPP4A gene:

To demonstrate the presence of novel splice variants of INPP4A gene expressed in blood of atopic asthmatic patients. RT PCR was carried out from the RNA of 12 atopic asthmatics and 4 normal healthy individuals. RNA was isolated from the total leukocytes using EZ-RNA isolation kit, following manufacturer's directions (Biological, Industries). cDNA was made from 10 µg of total RNA using cDNA Archive kit from Applied Biosystems. PCR was carried out in a total volume of 20 µl containing cDNA corresponding to 100 ng of starting RNA, 2.0 pmol each of forward and reverse primer (SEQ ID 25 and 26 respectively), 1.5Mm MgCl₂, 0.25mM of each dNTP, 0.03U/µl of Taq DNA polymerase (Bangalore Genie, India) and the buffer recommended by the supplier. PCR conditions: Denaturation at 94 degree C for 5 minutes, 30 cycles of denaturation at 94 degree C for 45 seconds, annealing at 65 degree C for 45 seconds, extension at 72 degree C for 45 seconds; followed by a 10 minutes 72 degree C segment extension period. To demonstrate the presence of novel splice variants of INPP4A gene expressed in blood of topic asthmatic patients. RT PCR was carried out from the RNA of 12 atopic asthmatics and 4 normal healthy individuals. Two additional splice variants apart from the reported α 3 were identified in four atopic asthmatic individuals, whereas no splice variant was observed in the normal healthy individuals (Figure 8).

### EXAMPLE 8

### Allergen induced expression of INPP4A protein profile:

The BALB/c mice were sensitized by intra-peritoneal injections of 20 µg chicken egg ovalbumin (OVA) (grade V ≥ 98% pure, Sigma Chemical Co., St. Louis, MO, USA) in 0.2 ml saline adsorbed on 2 mg alum (sensitized) or alum alone (control) on days 0, 7 and 14. Mice were then challenged with aerosolized 3% ovalbumin (sensitized) or aerosolized saline (control) 30 minutes per day for 10 consecutive days (from day 22 to day 32). Responsiveness to methacholine (Sigma-Aldrich) was assessed in conscious, unrestrained mice by barometric plethysmography, using protocol, apparatus and software supplied by BUXCO (Troy, NY, USA). Mice were sacrificed 16 hrs after the last challenge and lung tissue were taken for immunohistochemistry. Immunohistochemistry of mouse lung section was done using commercial goat polyclonal antibody sc-12315 (Santa Cruz Biotechnology, Inc.; CA, USA), raised against a peptide near the amino terminus of INPP4A of human and mouse origin following standard protocol with slight modifications (Ather MH et al, 2004). Goat gamma globulin was used as isotype control (Jackson Immunoresearch Laboratories, Inc.; PA, USA).

The INPP4A protein was seen to be expressed at lower levels in ovalbumin induced mouse model of asthma than in the saline treated controls (Figure 9).

### EXAMPLE 9

### Restoration of INPP4A protein by treatment with anti-inflammatory agent such as steroid:

The BALB/c mice were sensitized by intra-peritoneal injections of 20 µg chicken egg ovalbumin (OVA) (grade V ≥ 98% pure, Sigma Chemical Co., St. Louis, MO, USA) in 0.2 ml saline adsorbed on 2 mg alum on days 0, 7 and 14. Mice were then challenged with aerosolized 3% ovalbumin 30 minutes per day for 10 consecutive days (from day 22 to day 32). Treatment with 1 mg/kg dexamethasone was given starting from the day of last sensitization to last challenge (day 14-32). Bronchial hyperresponsiveness was measured as described previously using BUXCO plethysmograph (Troy, NY, USA). Mice were sacrificed 16 hrs after the last OVA challenge and lungs were subjected to immunohistochemistry. Immunohistochemistry of mouse lung section was done using commercial goat polyclonal antibody sc-12315 (Santa Cruz Biotechnology, Inc.; CA, USA), raised against a peptide near the amino terminus of *INPP4A* of human and mouse origin following standard protocol with slight modifications (Ather MH et al, 2004). Goat gamma globulin was used as isotype control (Jackson Immunoresearch Laboratories, Inc.; PA, USA). The levels of *INPP4A* protein were found to be restored to normal levels in case of steroid treated ovalbumin induced mouse model of asthma (Figure 9).

**Table 1: - Frequency (%) of SNPs in patients and controls.**

| SNP ID | Genotype | Patients (N= 192) | Controls (N= 272) | |
|---|---|---|---|---|
| +92031 A/T | TT | 3.19 | 7.78 | |
| (rs3769712) | AT | 32.45 | 38.15 | |
| | AA | 64.36 | 54.07 | 15 |
| +92344 C/T | TT | 0.54 | 1.13 | |
| (rs3769710) | CT | 13.98 | 19.17 | |
| | CC | 85.48 | 79.7 | |
| +92817 C/T | TT | 3.51 | 7.48 | 20 |
| (rs2278208) | CT | 33.92 | 37.4 | |
| | CC | 62.57 | 55.12 | |
| +110832 A/G | GG | 3.3 | 7.58 | |
| (rs2278206) | AG | 34.62 | 36.74 | 25 |
| | AA | 62.09 | 55.68 | |
| +131237 | C/T TT | 7.61 | 6.67 | |
| (rs10201079) | CT | 40.76 | 32.22 | |
| | CC | 51.63 | 61.11 | |

**Table 2: - Frequency (%) of haplotypes constructed using D2S311/rs37697i2/CA repeat/ rs2278206 markers in patients and controls estimated by PHASE. Haplotypes with relative frequencies > 0.025 (2.5 % of sample size) in either of the groups have been depicted below.**

| **S:NO.** | **HAPLOTYPE** | **PATIENTS** | **CONTROLS** |
|---|---|---|---|
| 1. | 398_A_154_A | 2.86 | 0.91 |
| 2. | 400_T_152_G | 9.11 | 12.32 |
| 3. | 400_A_154_A | 7.29 | 13.05 |
| 4. | 402_T_152_G | 1.04 | 3.49 |
| 5. | **402_A_154_A** | **16.14** | **4.96** |
| 6. | 404_T_152_G | 6.25 | 5.51 |
| 7. | 404_A_154_A | 45.57 | 40.26 |
| 8. | 406_A_154_A | 2.60 | 6.62 |

**Table 3: Transmission Disequilibrium Test in families (N= 158) for the eight loci studied.**

**Table 3 (a): D2S2311 /M1 (p= 0.0007)**

| **Allele** | **Transmitted** | **Non-transmitted** | **Chi-Sq** |
|---|---|---|---|
| 386 | 4 | 0 | 4 |
| 396 | 3 | 5 | 0.5 |
| 398 | 9 | 11 | 0.2 |
| 400 | 27 | 58 | 11.306 |
| 402 | 36 | 22 | 3.379 |
| 404 | 72 | 50 | 3.967 |
| 406 | 4 | 9 | 1.923 |
| 408 | 5 | 5 | 0 |

**Table 3 (b): D2S2187/M2 (p= 0.19)**

| **Allele** | **Transmitted** | **Non-transmitted** | **Chi-Sq** |
|---|---|---|---|
| 224 | 2 | 1 | 0.333 |
| 226 | 1 | 1 | 0 |
| 228 | 6 | 5 | 0.091 |
| 230 | 13 | 21 | 1.882 |
| 232 | 34 | 41 | 0.653 |
| 234 | 84 | 62 | 3.315 |
| 236 | 39 | 44 | 0.301 |
| 238 | 15 | 24 | 2.077 |
| 240 | 4 | 2 | 0.667 |
| 244 | 3 | 0 | 3 |

**Table 3 (c): rs3769712 / SS1 (p= 0.0027)**

| **Allele** | **Transmitted** | **Non-transmitted** | **Chi-Sq** |
|---|---|---|---|
| A | 65 | 35 | 9 |
| T | 35 | 65 | 9 |

**Table 3 (d): SS2 rs3769710 (p= 0.058)**

| **Allele** | **Transmitted** | **Non-transmitted** | **Chi-Sq** |
|---|---|---|---|
| C | 30 | 17 | 3.596 |
| T | 17 | 30 | 3.596 |

**Table 3 (e): rs2278208 / SS3 (p=0.063)**

| **Allele** | **Transmitted** | **Non-transmitted** | **Chi-Sq** |
|---|---|---|---|
| C | 56 | 38 | 3.447 |
| T | 38 | 56 | 3.447 |

**Table 3 (f): CA Repeat (p=0.008)**

| **Allele** | **Transmitted** | **Non-transmitted** | **Chi-Sq** |
|---|---|---|---|
| 152 | 38 | 65 | 7.078 |
| 154 | 65 | 38 | 7.078 |

**Table 3 (g): rs2278206 / SS4 (p= 0.0007)**

| **Allele** | **Transmitted** | **Non-transmitted** | **Chi-Sq** |
|---|---|---|---|
| A | 78 | 41 | 11.504 |
| G | 41 | 78 | 11.504 |

**Table 3 (h): rs10201079 / SS5 (p= 0.03)**

| **Allele** | **Transmitted** | **Non-transmitted** | **Chi-Sq** |
|---|---|---|---|
| C | 34 | 54 | 4.545 |
| T | 54 | 34 | 4.545 |

### SEQUENCE LISTING:

### General Information

### APPLICANT: CSIR, INDIA

TITLE OF INVESTIGATION: Genetic variants of human *INOSITOL POLYPHOSPHATE-4-PHOSPHATASE, TYPE I (INPP4A)* and diagnosis, prediction and prevention of immunological disorders including atopic asthma.

### NUMBER OF SEQUENCES: 26

i. **INFORMATION FOR SEQ ID No: 1**
ii. **SEQUENCE CHARACTERISTICS:** consists of CA dinucleotides (D2S2311) at nucleotide 31-73
iii. **LENGTH:** 401 bp
iv. **TYPE:** DNA
V.
vi. **ORGANISM:** Human (Natural sequence)
vii. **IMMEDIATE:** Natural sequence
viii. **NAME/KEY:** N.A
ix. **SEQUENCE ID** # 1

i. **INFORMATION FOR SEQ ID No:** 2
ii. **SEQUENCE CHARACTERISTICS:** consists of GT dinucleotides (D2S2187) at nucleotide 116-164
iii. **LENGTH:** 240 bases
iv. **TYPE:** DNA
v.
vi. **ORGANISM:** Human (Natural sequence)
vii. **IMMIDIATE:** Natural sequence
viii. **NAME/KEY:** N.A ix. **SEQUENCE ID** # 2

i. **INFORMATION FOR SEQ ID No:** 3
ii. **SEQUENCE CHARACTERISTICS:** consists of CA dinucleotides at nucleotide 105 to 122
iii. **LENGTH:** 159 bases
iv. **TYPE:** DNA
v.
vi. **ORGANISM:** Human (Natural sequence)
vii. **IMMIDIATE:** Natural sequence
viii. **NAME/KEY:** N.A
ix. **SEQUENCE ID** # 3

i. **INFORMATION FOR SEQ ID No:** 4
ii. **SEQUENCE CHARACTERISTICS:** consists of 75 A/T polymorphism (rs3769712), 388 C/T polymorphism (rs3769710) and 861 C/T (rs2278208 ) at nucleotide 229
iii. **LENGTH:** 1036 bases
iv. **TYPE:** DNA
v.
vi. **ORGANISM:** Human (Natural sequence)
vii. **IMMEDIATE:** Natural sequence
viii. **NAME/KEY:** N.A.
ix. **SEQUENCE ID** # 4

i. **INFORMATION FOR SEQ ID No:** 5
ii. **SEQUENCE CHARACTERISTICS:** Consists of G/A polymorphism (rs2278206) at nucleotide 147
iii. **LENGTH:** 961 bases
iv. **TYPE:** DNA
v.
vi. **ORGANISM:** Human (Natural sequence)
vii. **IMMIDIATE:** Natural sequence
viii. **NAME/KEY:** N.A
ix. **SEQUENCE ID** # 5

i. **INFORMATION FOR SEQ ID No:** 6
ii. **SEQUENCE CHARACTERISTICS:** Consists of C/T polymorphism (rs10201079) at nucleotide 1221
iii. **LENGTH:** 1707 bases
iv. **TYPE:** DNA
v.
vi. **ORGANISM:** Human (Natural sequence)
vii. **IMMEDIATE:** Natural sequence
viii. **NAME/KEY:** N.A.
ix. **SEQUENCE ID** # 6

i. **INFORMATION FOR SEQ ID No:** VII
ii. **SEQUENCE CHARACTERISTICS:** Forward primer for Sequence ID: 1
iii. **LENGTH:** 18 bases
iv. **TYPE:** DNA
v. **5'** GGG GCA AGT GGC GAT AGG **3'**
vi. **ORGANISM:** Human (Natural sequence)
vii. **IMMEDIATE:** Natural sequence
viii. **NAME/KEY:** Synthetic oligonucleotide
ix. **SEQUENCE ID** # 7

i. **INFORMATION FOR SEQ ID No:** VIII
ii. **SEQUENCE CHARACTERISTICS:** Reverse primer for Sequence ID: 1
iii. **LENGTH:** 20 bases
iv. **TYPE:** DNA
v. **5'** TGT TCC CCC ATG GCA GTG TA **3'**
vi. **ORGANISM:** Human (Natural sequence)
vii. **IMMIDIATE:** Natural sequence
viii. **NAME/KEY:** Synthetic oligonucleotide
ix. **SEQUENCE ID** # 8

i. **INFORMATION FOR SEQ ID No:** IX
ii. **SEQUENCE CHARACTERISTICS:** Forward primer for Sequence ID: 2
iii. **LENGTH:** 21 bases
iv. **TYPE: DNA**
v. **5'** GGG CTG GAG GGG GTG GAG TAG **3'**
vi. **ORGANISM:** Human (Natural sequence)
vii. **IMMIDIATE:** Natural sequence
viii. **NAME/KEY:** Synthetic oligonucleotide
ix. **SEQUENCE ID** # 9

i. **INFORMATION FOR SEQ ID No:** X
ii. **SEQUENCE CHARACTERISTICS:** Reverse primer for Sequence ID: 2
iii **LENGTH:** 25 bases
iv. **TYPE:** DNA
v. **5'** ATC ACC CCA CAG AGC TAA GGC AAC A **3'**
vi. **ORGANISM:** Human (Natural sequence)
vii. **IMMIDIATE:** Natural sequence
viii. **NAME/KEY:** Synthetic oligonucleotide
ix. **SEQUENCE ID** # 10

i. **INFORMATION FOR SEQ ID No:** XI
ii. **SEQUENCE CHARACTERISTICS:** Forward primer for Sequence ID: 3
iii. **LENGTH:** 23 bases
iv. **TYPE:** DNA
v. **5'** TCA CCC AGT ACC AGA CCA TCA TC **3'**
vi. **ORGANISM:** Human (Natural sequence)
vii. **IMMIDIATE:** Natural sequence
viii. **NAME/KEY:** Synthetic oligonucleotide
ix. **SEQUENCE ID** # 11

i. **INFORMATION FOR SEQ ID No:** XII
ii. **SEQUENCE CHARACTERISTICS:** Reverse primer for Sequence ID: 3
iii. **LENGTH:** 22 bases
iv. **TYPE:** DNA
v. **5'** TGG GAA AGC AGA GGG AGT GAC T **3'**
vi. **ORGANISM:** Human (Natural sequence)
vii. **IMMEDIATE:** Natural sequence
viii. **NAME/KEY:** Synthetic oligonucleotide
ix. **SEQUENCE ID** # 12

i. **INFORMATION FOR SEQ ID No:** XIII
ii. **SEQUENCE CHARACTERISTICS:** Forward primer for Sequence ID: 4
iii. **LENGTH:** 24 bases
iv. **TYPE:** DNA
v. **5'** TGC AGC AGC CCT TAG TTT GTT TAG **3'**
vi. **ORGANISM:** Human (Natural sequence)
vii. **IMMEDIATE:** Natural sequence
viii. **NAME/KEY:** Synthetic oligonucleotide
ix. **SEQUENCE ID** # 13

i. **INFORMATION FOR SEQ ID No:** XIV
ii. **SEQUENCE CHARACTERISTICS:** Reverse primer for Sequence ID: 4
iii. **LENGTH:** 18 bases
iv. **TYPE:** DNA
v. **5'** AGG GGG CCG TTG GTC TGA **3'**
vi. **ORGANISM:** Human (Natural sequence)
vii. **IMMIDIATE:** Natural sequence
viii. **NAME/KEY:** Synthetic oligonucleotide
ix. **SEQUENCE ID** # 14

i. **INFORMATION FOR SEQ ID No:** 15
ii **SEQUENCE CHARACTERISTICS:** Forward primer for Sequence ID: 5
iii **LENGTH:** 23 bases
iv **TYPE:** DNA
v **5'** ATG GCG AGG GCT GTG AGG ATG TC **3'**
vi **ORGANISM:** Human (Natural sequence)
vii **IMMIDIATE:** Natural sequence
viii **NAME/KEY:** Synthetic oligonucleotide
ix **SEQUENCE ID** # 15

i. **INFORMATION FOR SEQ ID No:** XVI
ii. **SEQUENCE CHARACTERISTICS:** Reverse primer for Sequence ID: 5
iii. **LENGTH:** 24 bases
iv. **TYPE:** DNA
v. **5'** GAA GCT AGG TCT GTG GGC AAG TCT **3'**
vi. **ORGANISM:** Human (Natural sequence)
vii. **IMMIDIATE:** Natural sequence
viii. **NAME/KEY:** Synthetic oligonucleotide
ix. **SEQUENCE ID** # 16

i. **INFORMATION FOR SEQ ID No:** 17
ii. **SEQUENCE CHARACTERISTICS:** Forward primer for Sequence ID: 6
iii. **LENGTH:** 21 bases
iv. **TYPE:** DNA
v. **5'** CCT TGG CTG CAT GGG TTC TCA **3'**
vi. **ORGANISM:** Human (Natural sequence)
vii. **IMMEDIATE:** Natural sequence
viii. **NAME/KEY:** Synthetic oligonucleotide
ix. **SEQUENCE ID** # 17

i. **INFORMATION FOR SEQ ID No:** 18
ii. **SEQUENCE CHARACTERISTICS:** Reverse primer for Sequence ID: 6
iii. **LENGTH:** 21 bases
iv. **TYPE:** DNA
v. **5'** CAT GCC TGG GCC CTC TTA TCA **3'**
vi. **ORGANISM:** Human (Natural sequence)
vii. **IMMEDIATE:** Natural sequence
viii. **NAME/KEY:** Synthetic oligonucleotide
ix. **SEQUENCE ID** # 18

i. **INFORMATION FOR SEQ ID No:** 19
ii. **SEQUENCE CHARACTERISTICS:** Snapshot primer for SNP 75 A/T (rs3769712) in Sequence ID: 4
iii. **LENGTH:** 19 bases
iv. **TYPE:** DNA
v. **5'** CAA AGC CCT TTC CCT TAC A **3' (Reverse strand)**
vi. **ORGANISM:** Human (Natural sequence)
vii. **IMMEDIATE:** Natural sequence
viii. **NAME/KEY:** Synthetic oligonucleotide
ix. **SEQUENCE ID** # 19

i. **INFORMATION FOR SEQ ID No:** 20
ii. **SEQUENCE CHARACTERISTICS:** Snapshot primer for 388 C/T polymorphism (rs3769710) in Sequence ID: 4
iii. **LENGTH:** 22 bases
iv. **TYPE:** DNA
v. **5'** ATC CCG AAG GGT GGC TGA TGG T **3' (Reverse strand)**
vi. **ORGANISM:** Human (Natural sequence)
vii. **IMMEDIATE:** Natural sequence
viii. **NAME/KEY:** Synthetic oligonucleotide
ix. **SEQUENCE ID** # 20

i. **INFORMATION FOR SEQ ID No:** 21
ii **SEQUENCE CHARACTERISTICS:** Snapshot primer for 861 C/T (rs2278208) in Sequence ID: 4
iii **LENGTH:** 25 bases
iv **TYPE:** DNA
v **5' AGC** CGT ACT GGG TCA TCA GGG TCC T **3' (Reverse strand)**
vi **ORGANISM:** Human (Natural sequence)
vii **IMMEDIATE:** Natural sequence
viii **NAME/KEY:** Synthetic oligonucleotide
ix **SEQUENCE ID** # 21

i. **INFORMATION FOR SEQ ID No:** XXII
ii. **SEQUENCE CHARACTERISTICS:** Snapshot primer for C/T polymorphism (rs2278206) at nucleotide 147 in Sequence ID: 5
iii. **LENGTH:** 20 bases
iv. **TYPE:** DNA
v. **5'** CAG ATG AGG ATG GCA TGC AG **3' (Reverse strand)**
vi. **ORGANISM:** Human (Natural sequence)
vii. **IMMIDIATE:** Natural sequence
viii. **NAME/KEY:** Synthetic oligonucleotide
ix. **SEQUENCE ID** # 22

i. **INFORMATION FOR SEQ ID No:** 23
ii. **SEQUENCE CHARACTERISTICS:** Snapshot primer for C/T polymorphism (rs10201079) at nucleotide 1221 in sequence ID: 6
iii **LENGTH:** 18 bases
iv **TYPE:** DNA
v **5'** GAT TGC CAC AGA TTT ACA **3'**
vi **ORGANISM:** Human (Natural sequence)
vii **IMMEDIATE:** Natural sequence
viii **NAME/KEY:** Synthetic oligonucleotide
ix **SEQUENCE ID** # 23

i. **INFORMATION FOR SEQ ID No:** 24
ii. **SEQUENCE CHARACTERISTICS:** CDNA sequence containing splice variant region
iii **LENGTH:** variable from 817 -598 nucleotides
iv **TYPE:** DNA
vi **ORGANISM:** Human (Natural sequence)
vii **IMMIDIATE:** Natural sequence
viii **NAME/KEY:** N.A.
ix **SEQUENCE ID** # 24

i. **INFORMATION FOR SEQ ID No:** 25
ii. **SEQUENCE CHARACTERISTICS:** Forward primer for CDNA sequence containing splice variant region
iii **LENGTH:** 19 bases
iv **TYPE:** DNA
v **5'** GGG GCG CCA GCA GCA CAC T **3'**
vi **ORGANISM:** Human (Natural sequence)
vii **IMMEDIATE:** Natural sequence
viii **NAME/KEY:** Synthetic oligonucleotide
ix **SEQUENCE ID** # 25

i. **INFORMATION FOR SEQ ID No:** 26
ii. **SEQUENCE CHARACTERISTICS:** Reverse primer for CDNA sequence containing splice variant region
iii **LENGTH:** 24 bases
iv **TYPE:** DNA
v **5'** GCG GGT AAA GGG CCT CAC TCC ATT **3'**
vi **ORGANISM:** Human (Natural sequence)
vii **IMMIDIATE:** Natural sequence
viii **NAME/KEY:** Synthetic oligonucleotide
ix **SEQUENCE ID** # 26

### References:

1. Abney M, Ober C, McPeek MS (2002) Quantitative-trait homozygosity and association mapping and empirical genomewide significance in large, complex pedigrees: fasting serum-insulin level in the Hutterites. Am J Hum Genet 70:920-34.
2. Alam R et al (1994) Transforming growth factor beta abrogates the effects of hematopoietins on eosinophils and induces their apoptosis. J Exp Med 179: 1041-1045.
3. Altshuler D, Kruglyak L, Lander E (1998) Genetic polymorphisms and disease. N Engl J Med 338:1626.
4. Ather MH, Abbas F, Faruqui N, Israr M, Pervez S. Expression of pS2 in prostate cancer correlates with grade and Chromogranin A expression but not with stage. BMC Urol. 2004; 4(1):14.
5. Aubert JD, Dalal BI, Bai TR et al (1994) Transforming growth factor β1 gene expression in human airways. Thorax 49:225-232.
6. Babron MC, Selinger-Leneman H, Dizier MH, Clerget-Darpoux F (2001) Homogeneity of asthma genome scan results. Genet Epidemiol 21 Suppl 1:S44-8.
7. Barringer KJ, Orgel L, Wahl G, Gingeras TR (1990) Blunt-end and single-strand ligations by Escherichia coli ligase: influence on an in vitro amplification scheme. Gene 89:117-22.
8. Baron M (2001) The search for complex disease genes: fault by linkage or fault by association? Mol Psychiatry 6:143-9.
9. Barnes PJ (2001) Th2 cytokines and asthma: an introduction. Respir Res 2:64-5.
10. Barnes KC (1999) Gene-environment and gene-gene interaction studies in the molecular genetic analysis of asthma and atopy. Clin Exp Allergy 29 Suppl 4:47-51.
11. Barnes KC, Neely JD, Duffy DL, Freidhoff LR, Breazeale DR, Schou C, Naidu RP, et al (1996) Linkage of asthma and total serum IgE concentration to markers on chromosome 12q: evidence from Afro-Caribbean and Caucasian populations. Genomics 37:41-50.
12. Barnes PJ. New aspects of asthma. J Intern Med 1992; 231:453-61
13. Ben-Asouli Y, Banai Y, Pel-Or Y, Shir A, Kaempfer R (2002) Human interferon-gamma mRNA autoregulates its translation through a pseudoknot that activates the interferon-inducible protein kinase PKR. Cell 108:221-32.
14. Bleecker ER, Postma DS, Meyers DA (1997) Evidence for multiple genetic susceptibility loci for asthma. Am J Respir Crit Care Med 156:S113-6.
15. Blumenthal MN, Amos DB (1987) Genetic and immunologic basis of atopic responses. Chest 91:176S-184S.
16. Blumenthal MN, Langefeld CD (2004) A genome-wide search for allergic response (atopy) genes in three ethnic groups: Collaborative Study on the Genetics of asthma. Hum Genet 114(2):157-164.
17. Blumenthal MN, Ober C (2004) Genome scan for loci linked to mite sensitivity: the Collaborative Study on the Genetics of Asthma (CSGA). Genes Immun 5(3):226-231.
18. Bodmer WF (1987) The human genome sequence and the analysis of multifactorial traits. Ciba Found Symp 130:215-28.
19. Breslow JL (1988) Apolipoprotein genetic variation and human disease. Physiol Rev 68:85-132.
20. Caraballo LR, Hernandez M (1990) HLA haplotype segregation in families with allergic asthma. Tissue Antigens 35:182-6.
21. Cargill M, Altshuler D, Ireland J, Sklar P, Ardlie K, Patil N, Shaw N, et al (1999) Characterization of single-nucleotide polymorphisms in coding regions of human genes. Nat Genet 22:231-8.
22. Carriere KC, Kochar SC(2000): Comparing sub-survival functions in a competing risks model.Lifetime Data Anal.: 6(1):85-97.
23. Chang C, Bowman JL, DeJohn AW, Lander ES, Meyerowitz EM (1988) Restriction fragment length polymorphism linkage map for Arabidopsis thaliana. Proc Natl Acad Sci USA 85:6856-60.
24. Christie L, Hine RJ, Parker JG, Burks W (2002). Food allergies in children affect nutrient intake and growth. J Am Diet Assoc.102 (11):1648-51.
25. Cohen D, Cohen O, Marcadet A, Massart C, Lathrop M, Deschamps I, Hors J, et al (1984) Class II HLA-DC beta-chain DNA restriction fragments differentiate among HLA-DR2 individuals in insulin-dependent diabetes and multiple sclerosis. Proc Natl Acad Sci U S A 81:1774-8.
26. Cookson WO, Young RP, Sandford AJ, Moffatt MF, Shirakawa T, Sharp PA, Faux JA, et al (1992) Maternal inheritance of atopic IgE responsiveness on chromosome 11q. Lancet 340:381-4.
27. Cookson W (1999) The alliance of genes and environment in asthma and allergy. Nature 402:B5-11.
28. Duffy DL (1997) Genetic epidemiology of asthma. Epidemiol Rev 19:129-43.
29. Elias JA, Zheng T, Lee CG, Homer RJ, Chen Q, Ma B, Blackburn M, Zhu Z. Transgenic Modeling of Interleukin-13 in the Lung. Chest. 2003 Mar;123(3 Suppl):339S-45S.
30. Erlich HA (eds), Freeman Press, N.Y., N.Y., 1992); PCR Protocols: A Guide to Methods and Applications (eds. Innis, et al., Academic Press, San Diego, Calif., 1990. ,
31. Friedhoff P, Hahn M, Wolfes H, Pingoud A (1993) Quantitative polymerase chain reaction with oligodeoxynucleotide ligation assay/enzyme-linked immunosorbent assay detection. Anal Biochem 215:9-16.
32. Fujii D, Brissenden J.E, Derynck R, Francke U (1986) Transforming growth factor beta gene maps to human chromosome 19 long arm and to mouse chromosome 7. Somat Cell Mol Genet 12:281-288
33. de Gouyon B, Melanitou E, Richard MF, Requarth M, Hahn IH, Guenet JL, Demenais F, et al (1993) Genetic analysis of diabetes and insulitis in an interspecific cross of the nonobese diabetic mouse with Mus spretus. Proc Natl Acad Sci U S A 90:1877-81.
34. Grainger DJ, Heathcote K, Chiano M, Sneider H, Kemp PR et al Genetic control of the circulating concentration of transforming growth factor β1. Hum Mol Gen 8:93-97
35. Hacia JG, Fan JB, Ryder O, Jin L, Edgemon K, Ghandour G, Mayer RA, et al (1999) Determination of ancestral alleles for human single-nucleotide polymorphisms using high-density oligonucleotide arrays. Nat Genet 22:164-7.
36. Herd CM, Page CP. Pulmonary immune cells in health and disease: platelets. Eur Respir J 1994; 7:1145-60
37. Hefferon WT, Groman DJ, Yurk EC, Garry RC (2004) A variable dinucleotide repeat in the CFTR gene contributes to phenotype diversity by forming RNA secondary structures that alter splicing. PNAS 101(10):3504-3509.
38. Heinzmann A, Grotherr P, Jerkic SP, Lichtenberg A, Braun S, Kruse S, Forster J, et al (2000) Studies on linkage and association of atopy with the chromosomal region 12q13-24. Clin Exp Allergy 30:1555-61.
39. Hirschhorn JN, Sklar P, Lindblad-Toh K, Lim YM, Ruiz-Gutierrez M, Bolk S, Langhorst B, et al (2000) SBE-TAGS: an array-based method for efficient single-nucleotide polymorphism genotyping. Proc Natl Acad Sci U S A 97:12164-9.
40. James E. Gem, Robert F. Lemanske, Jr. and William W. Busse (1999) Early life origins of asthma. J Clin Invest, 104:7, 837-843
41. Julier C, de Gouyon B, Georges M, Guenet JL, Nakamura Y, Avner P, Lathrop GM (1990) Minisatellite linkage maps in the mouse by cross-hybridization with human probes containing tandem repeats. Proc Natl Acad Sci U S A 87:4585-9.
42. Kameyoshi Y, Do" rchner A, Mallet AI, et al. (1992) RANTES released by thrombin-stimulated platelets is a potent attractant for human eosinophils. J Exp Med; 176:587-92
43. Kameyoshi Y, Schro" der JM, Christopher E, et al.( 1994) Identification of the cytokine RANTES released from platelets as an eosinophil chemotactic factor. Int Arch Allergy Immunol; 104(suppl 1):49-51
44. Klinger MH, Wilhelm D, Bubel S, et al.(1995) Immunocytochemical localization of the chemokines RANTES and MIP-1 alpha within human platelets and their release during storage. Int Arch Allergy Immunol; 107:541-4.6
45. Kreutz R, Hubner N, James MR, Bihoreau MT, Gauguier D, Lathrop GM, Ganten D, et al (1995) Dissection of a quantitative trait locus for genetic hypertension on rat chromosome 10. Proc Natl Acad Sci U S A 92:8778-82.
46. Kwoh DY, Davis GR, Whitfield KM, Chappelle HL, DiMichele LJ, Gingeras TR (1989) Transcription-based amplification system and detection of amplified human immunodeficiency virus type 1 with a bead-based sandwich hybridization format. Proc Natl Acad Sci U S A 86:1173-7.
47. Lander ES, Botstein D (1986) Strategies for studying heterogeneous genetic traits in humans by using a linkage map of restriction fragment length polymorphisms. Proc Natl Acad Sci U S A 83:7353-7.
48. Lander ES (1993) Finding similarities and differences among genomes. Nat Genet 4:5-6.
49. Lathrop GM, Lalouel JM, Julier C, Ott J (1984) Strategies for multilocus linkage analysis in humans. Proc Natl Acad Sci U S A 81:3443-6:
50. Levitt RC (1994) Molecular genetic methods for mapping disease genes. Am J Respir Crit Care Med 150:S94-9.
51. Longo DR, Johnson JC, Kruse RL, Brownson RC, Hewett JE (2001): A prospective investigation of the impact of smoking bans on tobacco cessation and relapse. Tob Control, Sep;10(3):267-72.
52. Mattila P, Korpela J, Tenkanen T, Pitkanen K (1991) Fidelity of DNA synthesis by the Thermococcus litoralis DNA polymerase--an extremely heat stable enzyme with proofreading activity. Nucleic Acids Res 19:4967-73.
53. Mokdad-Gargouri R, Belhadj K, Gargouri A (2001) Translational control of human p53 expression in yeast mediated by 5'-UTR-ORF structural interaction. Nucleic Acids Res 29:1222-7.
54. Mukawa A, Kamitsuma Y, Tsunekawa S, Tanaka N (1989): Report on a long-term trial of CYBEST Model 2 for prescreening for squamous cell carcinoma of the uterine cervix Anal Cell Pathol. Aug;1(4):225-33.
55. Nagarkatti R, B Rao C, Rishi JP, Chetiwal R, Shandilya V, Vijayan V, Kumar R, Pemde HK, Sharma SK, Sharma S, Singh AB, Gangal SV, Ghosh B (2002) Association of IFNG gene polymorphism with asthma in the Indian population. JACI 2002 Sep; 110(3):410-2.
56. Norris FA, Atkins RC, Majerus PW. (1997) Inositol polyphosphate 4-phosphatase is inactivated by calpain-mediated proteolysis in stimulated human platelets. J Biol Chem. Apr 25;272(17):10987-9
57. Ober C, Tsalenko A, Parry R, Cox NJ (2000) A second-generation genomewide screen for asthma-susceptibility alleles in a founder population. Am J Hum Genet 67:1154-62.
58. Oberle I, Heilig R, Moisan JP, Kloepfer C, Mattei GM, Mattei JF, Boue J, et al (1986) Genetic analysis of the fragile-X mental retardation syndrome with two flanking polymorphic DNA markers. Proc Natl Acad Sci U S A 83:1016-20.
59. Orita M, Iwahana H, Kanazawa H, Hayashi K, Sekiya T (1989) Detection of polymorphisms of human DNA by gel electrophoresis as single-strand conformation polymorphisms. Proc Natl Acad Sci U S A 86:2766-70.
60. Paul WE (1997) Interleukin 4: signalling mechanisms and control of T cell differentiation. Ciba Found Symp 204:208-16; discussion 216-9.
61. Piacquadio DJ, Chen DM, Farber HF, Fowler JF Jr, Glazer SD, Goodman JJ, Hruza LL, Jeffes EW, Ling MR, Phillips TJ, Rallis TM, Scher RK, Taylor CR, Weinstein GD (2004): Photodynamic therapy with aminolevulinic acid topical solution and visible blue light in the treatment of multiple actinic keratoses of the face and scalp: investigator-blinded, phase 3, multicenter trials. Arch Dermatol.; 140(1): 41-6.
62. Reich DE, Cargill M, Bolk S, Ireland J, Sabeti PC, Richter DJ, Lavery T, et al (2001) Linkage disequilibrium in the human genome. Nature 411:199-204.
63. Rothenburg S, Koch-Nolte F, Rich A, Haag F (2001) A polymorphic dinucleotide repeat in the rat nucleolin gene forms Z-DNA and inhibits promoter activity. Proc Natl Acad Sci U S A 98:8985-90.
64. Sachidanandam R, Weissman D, Schmidt SC, Kakol JM, Stein LD, Marth G, Sherry S, et al (2001) A map of human genome sequence variation containing 1.42 million single nucleotide polymorphisms. Nature 409:928-33.
65. Sambrook et al., Molecular Cloning, A Laboratory Manual, 2nd Ed., CSHP, New York 1989.
66. Saiki RK, Bugawan TL, Horn GT, Mullis KB, Erlich HA (1986) Analysis of enzymatically amplified beta-globin and HLA-DQ alpha DNA with allele-specific oligonucleotide probes. Nature 324:163-6.
67. Sham, P. C., and Curtis, D. (1995). "Monte Carlo tests for associations between disease and alleles at highly polymorphic loci." Annals of Human Genetics, 59, 97-105.
68. Shearn CT, Walker J, Norris FA. (2001) Identification of a novel spliceoform of inositol polyphosphate 4-phosphatase type Ialpha expressed in human platelets: structure of human inositol polyphosphate 4-phosphatase type Igene. Biochem Biophys Res Commun. Aug 10;286 (1):119-25.
69. Simon C. Pitchford (2004) Platelets are necessary for airway wall remodeling in a murine model of chronic allergic inflammation. Blood:103, 2
70. Sorensen G, Emmons K, Stoddard AM, Linnan L, Avrunin J (2002): Do social influences contribute to occupational differences in quitting smoking and attitudes toward quitting? Am J Health Promot. Jan-Feb;16(3):135-41.
71. Stephens M, Donnelly P (2003): A comparison of bayesian methods for haplotype reconstruction from population genotype data. Am J Hum Genet. Nov 73:1162-9.
72. Tay AH, Tan EC, Chew FT, Goh DL, Shek LP, Lee BW (1999) Ethnic differences in genetic susceptibility to atopy and asthma. Asian Pac J Allergy Immunol 17:239-42.
73. Thomas NS, Wilkinson J, Holgate ST (1997) The candidate region approach to the genetics of asthma and allergy. Am J Respir Crit Care Med 156:S144-51.
74. Vyas P, Norris FA, Joseph R, Majerus PW, Orkin SH. (2000) Inositol polyphosphate 4-phosphatase type I regulates cell growth downstream of transcription factor GATA-1. Proc Natl Acad Sci U S A. Dec 5;97 (25):13696-701
75. Xu J, Wiesch DG, Meyers DA (1998) Genetics of complex human diseases: genome screening, association studies and fine mapping. Clin Exp Allergy 28 Suppl 5:1-5; discussion 26-8.
76. Xu J, Meyers DA, Ober C, Blumenthal MN, Mellen B, Barnes KC, King RA, et al (2001) Genomewide screen and identification of gene-gene interactions for asthma-susceptibility loci in three U.S. populations: collaborative study on the genetics of asthma. Am J Hum Genet 68:1437-46.

## Claims

1. An *in vitro* method of predicting susceptibility of an individual to asthma which comprises determining whether said individual possesses a haplotype of genetic variants, said variants being selected from genetic variants of the human Inositol polyphosphate 4-phosphatase (INPP4A) gene and the CA dinucleotide repeat at the M1 locus located 44.7 kb upstream of the start site of said gene, wherein said haplotype is positively-associated or negatively-associated with asthma occurrence.

2. A method as claimed in claim 1, wherein a haplotype is determined representing a combination of variants selected from an allelic variant of said M1 locus CA dinucleotide repeat, +92031A/T S1 locus variant, M3 locus CA dinucleotide repeat at +99095 and +110832 A/G S4 locus variant.

3. A method as claimed in claim 2 wherein a four-locus haplotype is determined with reference in order to the loci M1_S1_M3_S4 and selected from:
| | | | |
|---|---|---|---|
| 396_T_154_G, | 398_A_152_A, | 400_T_152_A, | 400_A_152_A, |
| 406_T_152_A, | 406_A_156_T, | 412_A_154_A, | 400_T_154_A, |
| 402_T_152_A, | 404_A_156_T, | 410_A_154_A, | 404_A_152_G, |
| 406_A_152_A, | 404_A_152_A, | 406_T_152_G, | 396_A_152_G, |
| 400_A_154_G, | 402_T_154_A, | 402_A_152_A, | 404_T_154_A, |
| 400_T_154_G, | 396_T_152_G, | 404_T_152_A, | 398_A_152_G, |
| 386_A_154_A, | 402_T_152_G, | 398_T_152_G, | 404_A_154_G, |
| 408_A_154_A, | 406_A_154_A, | 398_A_154_A, | 404_T_152_G, |
| 400_A_154_A, | 400_T_152_G, | 402_A_154_A, | 404_A_154_A |
wherein the first number represents the length in base pairs of the fragment obtainable by PCR amplification corresponding to the M1 locus using the primer pair SEQ. ID nos. 7 and 8 and the last number represents the length in base pairs obtainable by PCR amplification corresponding to the M3 locus using the primer pair SEQ. ID nos. 11 and 12.

4. A method as claimed in claim 3, wherein the haplotype 402_A_154_A is detected as a risk factor for asthma.

5. A method as claimed in claim 3, wherein the haplotype 400_A_154_A and/or 400_T_152_G is/are detected as negatively associated with occurrence of asthma and/or the haplotype 400_T_152_G is detected as negatively associated with occurrence of atopic asthma.

6. Primers defined by SEQ ID Nos. 7-18 for use in predicting predisposition to asthma.

7. Primers according to claim 6 further supplemented with the primers defined by SEQ. ID Nos. 19-23.

8. Use in vitro of primers defined by SEQ ID Nos. 7-18 for predicting predisposition to asthma.

9. The use according to claim 8 wherein said primers are further supplemented by the primers defined by SEQ. ID Nos. 19-23.

10. An in vitro method for detecting and predicting predisposition to asthma by screening for INPP4A haplotypes and splice variants and their expression in a subject, the said method comprising the steps of:
[a] isolating DNA from samples selected from whole blood, semen, saliva, tears, urine, fecal material, sweat, buccal, skin or hair;
[b] providing primers having SEQ. ID nos. 7-18;
[c] amplifying and sequencing genomic DNA stretches using primers of SEQ ID Nos. 7 - 18, wherein primers of SEQ ID Nos. 7, 9, 11, 13, 15, 17 are forward primers and primers of SEQ ID Nos. 8, 10, 12, 14, 16, 18 are reverse primers by:
[d] amplifying and sequencing the DNA stretch of SEQ ID No. I of the INPP4A gene using the primer combination of SEQ ID Nos. 7 and 8, SEQ. ID No.1 containing sequence corresponding to the microsatellite repeat at the M1 locus D2S2311;
[e] amplifying and sequencing the DNA stretch of SEQ ID No. 2 of the INPP4A gene using the primer combination of SEQ ID Nos. 9 and 10, SEQ. ID No.2 containing sequence corresponding to the microsatellite repeat at the M2 locus D2S2187 at position +43987 intron 1;
[f] amplifying and sequencing the DNA stretch of SEQ ID No. 3 of the INPP4A gene using the primer combination of SEQ ID Nos. 11 and 12, SEQ. ID No. 3 containing sequence corresponding to the microsatellite repeat at the M3 locus +99095;
[g] amplifying and sequencing the DNA stretch of SEQ ID No. 4 of the INPP4A gene using the primer combination of SEQ ID Nos. 13 and 14, SEQ. ID No. 4 containing the single nucleotide polymorphism (SNP) sites S1 locus +92031A/T, S2 locus +92344 C/T and S3 locus +92817C/T;
[h] amplifying and sequencing the DNA stretch of SEQ ID No.5 of the INPP4A gene using the primer combination of SEQ ID Nos. 15 and 16, SEQ. ID. No. 5 containing the SNP site at the S4 locus at position +110832A/G in splice variant exon17;
[i] amplifying and sequencing the DNA stretch of SEQ ID No.6 of the INPP4A gene using the primer combination of SEQ ID Nos. 17 and 18, SEQ. ID No. 6 containing the SNP site at the S5 locus at position +131237 C/T, and
[j] validating and identifying the specific INPP4A gene variants computationally by comparison with the known wild type INPP4A gene sequences; and
[k] isolating RNA from whole blood samples; and
[l] isolating and identifying splice variants of SEQ ID No. 24 using the primer combination of SEQ ID Nos. 25 and 26.

11. A methods as claimed in claim 10, wherein the subject is human.

12. Pharmacogenetic markers having SEQ ID Nos. 1, 2, 3, 4, 5, 6 and 24 for use in detecting and predicting predisposition to asthma in a subject, said markers having the following characteristics:
(i) the SEQ ID No. 1 contains 1-230 contiguous nucleotides containing a group of CA dinucleotides of locus MI present 44.7 kb upstream of the gene start site;
(ii) the SEQ ID No. 2 contains 1-400 contiguous nucleotides containing GT dinucleotides at locus M2;
(iii) the SEQ ID No. 3 has 1-159 contiguous nucleotides containing a CA repeat polymorphism at nucleotide 229 of locus M3;
(iv) the SEQ ID No. 4 has 1-1036 contiguous nucleotides containing an A/T polymorphism at nucleotide 75 of locus SI, a C/T polymorphism at nucleotide 388 of locus S2 and a C/T polymorphism at nucleotide 861 of locus S3;
(v) the SEQ ID No. 5 has 1-961 contiguous nucleotides containing a G/A polymorphism at nucleotide 147 of locus S4;
(vi) the SEQ ID No. 6 has 1-1707 contiguous nucleotides containing a C/T polymorphism at nucleotide 1221 of locus S5;
(vii) the SEQ ID No. 24 has 1-817 contiguous nucleotides containing splice variants.

13. A diagnostic kit comprising pharmacogenetic markers having nucleotides of SEQ ID Nos. 1, 2, 3, 4, 5, 6 and 24 along with an instruction manual for detecting and predicting predisposition to asthma in a subject.

14. Use in vitro of a diagnostic kit as claimed in claim 13 for predicting and detecting humans susceptible to asthma.

## Patentansprüche

1. *In-vitro*-Verfahren zum Vorhersagen der Anfälligkeit eines Individuums für Asthma, welches umfasst, zu bestimmen, ob das Individuum einen Haplotyp von genetischen Varianten besitzt, wobei die Varianten aus genetischen Varianten des humanen Inositolpolyphosphat-4-Phosphatase-(INPP4A)-Gens und der CA-Dinucleotid-Wiederholung an dem M1-Locus, gelegen 44,7 kb stromaufwärts von der Startstelle des Gens, ausgewählt sind, wobei der Haplotyp mit dem Auftreten von Asthma positiv assoziiert oder negativ assoziiert ist.

2. Verfahren nach Anspruch 1, wobei ein Haplotyp bestimmt wird, der eine Kombination von Varianten, ausgewählt aus einer allelischen Variante der M1-Locus-CA-Dinucleotid-Wiederholung, +92031A/T-S1-Locus-Variante, M3-Locus-CA-Dinucleotid-Wiederholung bei +99095 und +110832A/G-S4-Locus-Variante, darstellt.

3. Verfahren nach Anspruch 2, wobei ein vier-Locus-Haplotyp mit Bezug in der Reihenfolge auf die Loci M1_S1_M3_S4 bestimmt und ausgewählt ist aus:
| | | | |
|---|---|---|---|
| 396_T_154_G, | 398_A_152_A, | 400_T_152_A, | 400_A_152_A, |
| 406_T_152_A, | 406_A_156_T, | 412_A_154_A, | 400_T_154_A, |
| 402_T_152_A, | 404_A_156_T, | 410_A_154_A, | 404_A_152_G, |
| 406_A_152_A, | 404_A_152_A, | 406_T_152_G, | 396_A_152_G, |
| 400_A_154_G, | 402_T_154_A, | 402_A_152_A, | 404_T_154_A, |
| 400_T_154_G, | 396_T_152_G, | 404_T_152_A, | 398_A_152_G, |
| 386_A_154_A, | 402_T_152_G, | 398_T_152_G, | 404_A_154_G, |
| 408_A_154_A, | 406_A_154_A, | 398_A_154_A, | 404_T_152_G, |
| 400_A_154_A, | 400_T_152_G, | 402_A_154_A, | 404_A_154_A, |
wobei die erste Zahl die Länge in Basenpaaren des Fragments, erhältlich durch PCR-Amplifikation entsprechend dem M1-Locus unter Verwendung des Primer-Paares SEQ. ID Nos. 7 und 8, darstellt und die letzte Zahl die Länge in Basenpaaren, erhältlich durch PCR-Amplifikation entsprechend dem M3-Locus unter Verwendung des Primer-Paares SEQ. ID Nos. 11 und 12, darstellt.

4. Verfahren nach Anspruch 3, wobei der Haplotyp 402_A_154_A als ein Risikofaktor für Asthma nachgewiesen wird.

5. Verfahren nach Anspruch 3, wobei der Haplotyp 400_A_154_A und/oder 400_T_152_G als negativ assoziiert mit dem Auftreten von Asthma nachgewiesen wird/werden und/oder der Haplotyp 400_T_152_G als negativ assoziiert mit dem Auftreten von atopischem Asthma nachgewiesen wird.

6. Primer, definiert durch die SEQ ID Nos. 7-18 zur Verwendung beim Vorhersagen einer Prädisposition für Asthma.

7. Primer gemäß Anspruch 6, weiter ergänzt mit den Primern, definiert durch die SEQ ID Nos. 19-23.

8. Verwendung *in vitro* von Primern, definiert durch die SEQ ID Nos. 7-18, zum Vorhersagen einer Prädisposition für Asthma.

9. Verwendung gemäß Anspruch 8, wobei die Primer weiter mit den Primern, definiert durch die SEQ ID Nos. 19-23, ergänzt sind.

10. *In-vitro*-Verfahren zum Nachweisen und Vorhersagen einer Prädisposition für Asthma durch Screenen für INPP4A-Haplotypen und Spleißvarianten und ihre Expression bei einem Patienten, wobei das Verfahren die Schritte umfasst:
(a) Isolieren von DNA aus Proben, ausgewählt aus Vollblut, Samen, Speichel, Tränen, Urin, fäkalem Material, Schweiß, Wange, Haut oder Haar;
(b) Bereitstellen von Primern mit den SEQ ID Nos. 7-18;
(c) Amplifizieren und Sequenzieren von genomischen DNA-Abschnitten unter Verwendung von Primern der SEQ ID Nos. 7-18, wobei die Primer der SEQ ID Nos. 7, 9, 11, 13, 15, 17 Vorwärtsprimer sind und die Primer der SEQ ID Nos. 8, 10, 12, 14, 16, 18 Rückwärtsprimer sind, durch:
(d) Amplifizieren und Sequenzieren des DNA-Abschnitts von SEQ ID No. 1 des INPP4A-Gens unter Verwendung der Primer-Kombination der SEQ ID Nos. 7 und 8, wobei SEQ ID No. 1 eine Sequenz entsprechend der Mikrosatellitenwiederholung an dem M1-Locus D2S2311 enthält;
(e) Amplifizieren und Sequenzieren des DNA-Abschnitts von SEQ ID No. 2 des INPP4A-Gens unter Verwendung der Primer-Kombination der SEQ ID Nos. 9 und 10, wobei SEQ ID No. 2 eine Sequenz entsprechend der Mikrosatellitenwiederholung an dem M2-Locus D2S2187 an der Position +43987 Intron 1 enthält;
(f) Amplifizieren und Sequenzieren des DNA-Abschnitts von SEQ ID No. 3 des INPP4A-Gens unter Verwendung der Primer-Kombination der SEQ ID Nos. 11 und 12, wobei SEQ ID No. 3 eine Sequenz entsprechend der Mikrosatellitenwiederholung an dem M3-Locus +99095 enthält;
(g) Amplifizieren und Sequenzieren des DNA-Abschnitts von SEQ ID No. 4 des INPP4A-Gens unter Verwendung der Primer-Kombination der SEQ ID Nos. 13 und 14, wobei SEQ ID No. 4 die Einzel-Nucleotid-Polymorphismus-(SNP)-Stellen S1-Locus +92031A/T, S2-Locus +92344 C/T und S3-Locus +92817C/T enthält;
(h) Amplifizieren und Sequenzieren des DNA-Abschnitts von SEQ ID No. 5 des INPP4A-Gens unter Verwendung der Primer-Kombination der SEQ ID Nos. 15 und 16, wobei SEQ ID No. 5 die SNP-Stelle an dem S4-Locus an Position +110832A/G in der Spleißvariante Exon17 enthält;
(i) Amplifizieren und Sequenzieren des DNA-Abschnitts von SEQ ID No. 6 des INPP4A-Gens unter Verwendung der Primer-Kombination der SEQ ID Nos. 17 und 18, wobei SEQ ID No. 6 die SNP-Stelle an dem S5-Locus an Position +131237C/T enthält, und
(j) Validieren und Identifizieren der spezifischen INPP4A-Genvarianten rechnerisch durch Vergleich mit den bekannten Wildtyp-INPP4A-Gensequenzen; und
(k) Isolieren von RNA aus Vollblutproben; und
(l) Isolieren und Identifizieren von Spleißvarianten von SEQ ID No. 24 unter Verwendung der Primer-Kombination der SEQ ID Nos. 25 und 26.

11. Verfahren nach Anspruch 10, wobei der Patient ein Mensch ist.

12. Pharmakogenetische Marker mit den SEQ ID Nos. 1, 2, 3, 4, 5, 6 und 24 zur Verwendung beim Nachweisen und Vorhersagen einer Prädisposition für Asthma bei einem Patienten, wobei die Marker die folgenden charakteristischen Eigenschaften haben:
(i) die SEQ ID No. 1 enthält 1-230 zusammenhängende Nucleotide, enthaltend eine Gruppe von CA-Dinucleotiden des Locus M1, vorhanden 44,7 kb stromaufwärts von der Gen-Startstelle;
(ii) die SEQ ID No. 2 enthält 1-400 zusammenhängende Nucleotide, enthaltend GT-Dinucleotide an Locus M2;
(iii) die SEQ ID No. 3 hat 1-159 zusammenhängende Nucleotide, enthaltend einen CA-Wiederholungs-Polymorphismus an Nucleotid 229 von Locus M3;
(iv) die SEQ ID No. 4 hat 1-1036 zusammenhängende Nucleotide, enthaltend einen A/T-Polymorphismus an Nucleotid 75 von Locus S1, einen C/T-Polymorphismus an Nucleotid 388 von Locus S2 und einen C/T-Polymorphismus an Nucleotid 861 von Locus S3;
(v) die SEQ ID No. 5 hat 1-961 zusammenhängende Nucleotide, enthaltend einen G/A-Polymorphismus an Nucleotid 147 von Locus S4;
(vi) die SEQ ID No. 6 hat 1-1707 zusammenhängende Nucleotide, enthaltend einen C/T-Polymorphismus an Nucleotid 1221 von Locus S5;
(vii) die SEQ ID No. 24 hat 1-817 zusammenhängende Nucleotide, enthaltend Spleißvarianten.

13. Diagnostischer Kit, umfassend pharmakogenetische Marker mit Nucleotiden der SEQ ID Nos. 1, 2, 3, 4, 5, 6 und 24 zusammen mit einer Bedienungsanleitung, zum Nachweisen und Vorhersagen einer Prädisposition für Asthma bei einem Patienten.

14. Verwendung *in vitro* von einem diagnostischen Kit nach Anspruch 13 zum Vorhersagen und Nachweisen von Menschen anfällig für Asthma.

## Revendications

1. Procédé *in vitro* de prédiction de la prédisposition d'un individu à l'asthme, lequel comprend déterminer si ledit individu possède un haplotype de variantes génétiques, lesdites variantes étant sélectionnées parmi des variantes génétiques du gène humain d'inositol polyphosphate-4-phosphatase (INPP4A) et la répétition du dinucléotide CA au locus M1 situé à 44,7 kb en amont du site de début dudit gène, où ledit haplotype est associé positivement ou associé négativement à l'occurrence d'asthme.

2. Procédé selon la revendication 1, dans lequel un haplotype est déterminé représentant une combinaison de variantes sélectionnées parmi une variante allèle de ladite répétition du dinucléotide CA au locus M1, une variante +92031 A/T au locus S1, la répétition du dinucléotide CA au locus M3 à +99095 et une variante +110832 A/G au locus S4.

3. Procédé selon la revendication 2, dans lequel un quatre-locus haplotype est déterminé par référence dans l'ordre aux locus M1_S1_M3_S4 et sélectionné parmi:
| | | | |
|---|---|---|---|
| 396_T_154_G, | 398_A_152_A, | 400_T_152_A, | 400_A_152_A, |
| 406_T_152_A, | 406_A_156_T, | 412_A_154_A, | 400_T_154_A, |
| 402_T_152_A, | 404_A_156_T, | 410_A_154_A, | 404_A_152_G, |
| 406_A_152_A, | 404_A_152_A, | 406_T_152_G, | 396_A_152_G, |
| 400_A_154_G, | 402_T_154_A, | 402_A_152_A, | 404_T_154_A, |
| 400_T_154_G, | 396_T_152_G, | 404_T_152_A, | 398_A_152_G, |
| 386_A_154_A, | 402_T_152_G, | 398_T_152_G, | 404_A_154_G, |
| 408_A_154_A, | 406_A_154_A, | 398_A_154_A, | 404_T_152_G, |
| 400_A_154_A, | 400_T_152_G, | 402_A_154_A, | 404_A_154_A, |
où le premier nombre représente la longueur en paires de bases du fragment qui peut être obtenu par amplification par PCR correspondant au locus M1 en utilisant la paire d'amorces des SEQ ID NO: 7 et 8 et le dernier nombre représente la longueur en paires de bases qui peut être obtenue par amplification par PCR correspondant au locus M3 en utilisant la paire d'amorces des SEQ ID NO: 11 et 12.

4. Procédé selon la revendication 3, dans lequel l'haplotype 402_A_154_A est détecté comme un facteur de risque pour l'asthme.

5. Procédé selon la revendication 3, dans lequel l'haplotype/les haplotypes 400_A_154_A et/ou 400_T_152_G est/sont détecté(s) comme négativement associé(s) à l'occurrence d'asthme et/ou l'haplotype 400_T_152_G est détecté comme négativement associé à l'occurrence d'asthme atopique.

6. Amorces définies par les SEQ ID NO: 7-18, à utiliser dans la prédiction d'une prédisposition à l'asthme.

7. Amorces selon la revendication 6, complétées en plus par les amorces définies par les SEQ ID NO: 19-23.

8. Utilisation *in vitro* des amorces définies par les SEQ ID NO: 7-18 pour prédire une prédisposition à l'asthme.

9. Utilisation selon la revendication 8, dans laquelle lesdites amorces sont encore complétées par les amorces définies par les SEQ ID NO: 19-23.

10. Procédé *in vitro* de détection et de prédiction d'une prédisposition à l'asthme, en criblant pour des haplotypes et des variantes d'épissage d'INPP4A et pour leur expression chez un sujet, ledit procédé comprenant les étapes consistant à:
[a] isoler l'ADN d'échantillons sélectionnés parmi le sang complet, le sperme, la salive, les larmes, l'urine, les matières fécales, la sueur, de la bouche, de la peau ou de poil;
[b] fournir des amorces ayant les SEQ ID NO: 7-18;
[c] amplifier et séquencer des sections d'ADN génomique en utilisant des amorces des SEQ ID NO: 7-18, où les amorces des SEQ ID NO: 7, 9, 11, 13, 15 et 17 sont des amorces sens et les amorces des SEQ ID NO: 8, 10, 12, 14, 16 et 18 sont des amorces anti-sens;
[d] amplifier et séquencer la section d'ADN de la SEQ ID NO: 1 du gène d'INPP4A en utilisant la combinaison d'amorces des SEQ ID NO: 7 et 8, la SEQ ID NO: 1 contenant la séquence correspondant à la répétition microsatellite au locus M1 D2S2311;
[e] amplifier et séquencer la section d'ADN de la SEQ ID NO: 2 du gène d'INPP4A en utilisant la combinaison d'amorces des SEQ ID NO: 9 et 10, la SEQ ID NO: 2 contenant la séquence correspondant à la répétition microsatellite au locus M2 D2S2187 en position +43987 à l'intron 1;
[f] amplifier et séquencer la section d'ADN de la SEQ ID NO: 3 du gène d'INPP4A en utilisant la combinaison d'amorces des SEQ ID NO: 11 et 12, la SEQ ID NO: 3 contenant la séquence correspondant à la répétition microsatellite au locus M3 +99095;
[g] amplifier et séquencer la section d'ADN de la SEQ ID NO: 4 du gène d'INPP4A en utilisant la combinaison d'amorces des SEQ ID NO: 13 et 14, la SEQ ID NO: 4 contenant le locus S1 +92031 A/T, le locus S2 +92344 C/T et le locus S3 +92817 C/T des sites de polymorphisme d'un seul nucléotide (SNP);
[h] amplifier et séquencer la section d'ADN de la SEQ ID NO: 5 du gène d'INPP4A en utilisant la combinaison d'amorces des SEQ ID NO: 15 et 16, la SEQ ID NO: 5 contenant le site SNP au locus S4 à la position +110832 A/G dans la variante d'épissage à l'exon 17;
[i] amplifier et séquencer la section d'ADN de la SEQ ID NO: 6 du gène d'INPP4A en utilisant la combinaison d'amorces des SEQ ID NO: 17 et 18, la SEQ ID NO: 6 contenant le site SNP au locus S5 à la position +131237 C/T, et
[j] valider et identifier les variantes spécifiques du gène d'INPP4A par calcul, par comparaison avec les séquences connues du gène INPP4A de type sauvage; et
[k] isoler l'ARN des échantillons de sang complet; et
[l] isoler et identifier les variantes d'épissage de la SEQ ID NO: 24 en utilisant la combinaison d'amorces des SEQ ID NO: 25 et 26.

11. Procédé selon la revendication 10, où le sujet est humain.

12. Marqueurs pharmacogénétiques ayant les SEQ ID NO: 1, 2, 3, 4, 5, 6 et 24, à utiliser dans la détection et la prédiction d'une prédisposition à l'asthme chez un sujet, lesdits marqueurs ayant les caractéristiques suivantes:
(i) la SEQ ID NO: 1 contient 1-230 nucléotides contigus contenant un groupe de dinucléotides CA du locus M1 présent 44,7 kb en amont du site de début du gène;
(ii) la SEQ ID NO: 2 contient 1-400 nucléotides contigus contenant des dinucléotides GT au locus M2;
(iii) la SEQ ID NO: 3 a 1-159 nucléotides contigus contenant un polymorphisme de répétition CA au nucléotide 229 du locus M3;
(iv) la SEQ ID NO: 4 a 1-1036 nucléotides contigus contenant un polymorphisme A/T au nucléotide 75 du locus S1, un polymorphisme C/T au nucléotide 388 du locus S2 et un polymorphisme C/T au nucléotide 861 du locus S3;
(v) la SEQ ID NO: 5 a 1-961 nucléotides contigus contenant un polymorphisme G/A au nucléotide 147 du locus S4;
(vi) la SEQ ID NO: 6 a 1-1707 nucléotides contigus contenant un polymorphisme C/T au nucléotide 1221 du locus S5;
(vii) la SEQ ID NO: 24 a 1-817 nucléotides contigus contenant des variantes d'épissage.

13. Kit de diagnostic comprenant des marqueurs pharmacogénétiques ayant des nucléotides des SEQ ID NO: 1, 2, 3, 4, 5, 6 et 24 et un manuel d'instructions pour détecter et prédire une prédisposition à l'asthme chez un sujet.

14. Utilisation *in vitro* d'un kit de diagnostic selon la revendication 13, pour prédire et détecter des êtres humains prédisposés à l'asthme.
